# EUROPEAN PATENT SPECIFICATION

(11) **EP 4 346 424 B1**
(45) Date of publication and mention of the grant of the patent: **22.07.2026**
(21) Application number: 22732074.4
(22) Date of filing: 31.05.2022
(51) Int. Cl.: A23C 19/02, A23J 3/10, C07K 14/47, C12N 1/06, C12P 21/00

(54) **METHOD FOR PRODUCING CASEIN**
VERFAHREN ZUR HERSTELLUNG VON KASEIN
PROCÉDÉ DE PRODUCTION DE CASÉINE

(30) Priority: 01.06.2021 EP 21305736; 18.09.2021 EP 21306294; 16.03.2022 EP 22305313
(43) Date of publication of application: 10.04.2024
(73) Proprietor: Standing Ovation, 75014 Paris (FR)
(72) Inventor: CHAYOT, Romain, 75014 PARIS (FR); DIALLO, Mamou, 91210 DRAVEIL (FR); LORILLIÈRE, Marion, 91280 SAINT PIERRE DU PERRAY (FR)
(74) Representative: Flesselles, Bruno F.G.
(86) International application number: PCT/EP2022/064728
(87) International publication number: WO 2022/253816

(56) References cited:
- WO-A1-2020/223700
- WO-A1-99/54355
- US-A- 4 519 945
- US-A- 4 550 028
- A.E. POST ET AL: "Effect of temperature and pH on the solubility of caseins: Environmental influences on the dissociation of S- and -casein", JOURNAL OF DAIRY SCIENCE, vol. 95, no. 4, 1 April 2012 (2012-04-01), US, pages 1603 - 1616, XP055627575, ISSN: 0022-0302, DOI: 10.3168/jds.2011-4641

## Description

The invention pertains to the food industry and relates to new methods for producing casein compositions and uses thereof, in particular for producing cheese substitute (notably vegan cheese).

### Background

The use of milk as a protein, sugar and lipid-rich nutrient, has become almost universal in traditional societies. In addition, milk transformation into various derivatives, is one of the most ancient examples of human agro-industries. A large diversity of cheese was elaborated all over the planet, becoming in certain countries a cultural benchmark, and being used not only as such, but also as an ingredient in many dishes, with the use of cheese in pizzas, burgers, or as additives for pastas being as many examples of international standards. Today, the cheese market represents 20 million tons of product per year, for a value of about $140bn.

However, dairy products, are associated with several issues or concerns, in terms of health, as well as environmental and ethical considerations. These concerns have highlighted the need for dairy product substitutes alleviating these various issues. Health issues include lactose intolerances, allergies (Mousan and Kamat (2016) Cow's Milk Protein Allergy. Clin Pediatr (Phila), Volume 55(11) pages 1054-63.; Manuyakorn and Tanpowpong (2018) Cow milk protein allergy and other common food allergies and intolerances. Paediatr Int Child Health, Volume 39(1), pages 32-40), and others, such as a high content in saturated fatty acids, known to have a potential negative impact on health. (Micha and Mozaffarian (2010) Saturated fat and cardiometabolic risk factors, coronary heart disease, stroke, and diabetes: A fresh look at the evidence. Lipids, Volume 45, pages 893-905; Jakobsen et al (2009) Major types of dietary fat and risk of coronary heart disease: A pooled analysis of 11 cohort studies. Am. J. Clin. Nutr; Volume 89, pages 1425-1432; Nettleton et al (2017) Saturated fat consumption and risk of coronary heart disease and ischemic stroke: A science update. Ann Nutr Metab Volume 70, pages 26-33). Lactose intolerance is due to the lack of lactase, the enzyme that degrades lactose in the stomach and small intestine, and result in an accumulation of lactose in the colon, and its digestion by bacteria (Ugidos-Rodriguez et al (2018) Lactose malabsorption and intolerance: a review. Food Funct, Volume 9(8), pages 4056-4068). It is a very common feature in humans, is genetically determined, and results in the need for a dairy-free diet. In a dairy product substitute, this composition could be modulated, to avoid lactose, and saturated fatty acids, and even, allergenic proteins.

In addition, environmental and ethical concerns related to food products of animal origin have raised over the last decades. The burden of animal breeding for a 7 billion population (11 billion in 2050) has become increasingly high. The environmental consequences are severe. They are today mostly considered in terms of anthropogenic greenhouse gas (GHG) emissions, water consumption, pollution by effluents, and land occupation.

Cattle breeding is today considered as one of the first sources GHG emissions, with an estimated 7.1 Gigatonnes of CO2-equivalent per year, representing 14,5% of all anthropogenic greenhouse gas (GHG) emissions the sources and methods (Rotz (2017) Modeling greenhouse gas emissions from dairy farms. J Dairy Science, Volume 101, pages 6675-6690), but a study from the UN estimated in 2010 the contribution of the global dairy sector alone to 4% of anthropogenic GGHG emissions (FOOD AND AGRICULTURE ORGANIZATION OF THE UNITED NATIONs, Greenhouse Gas Emissions from the Dairy Sector. A Life Cycle Assessment).

A very strong water consumption is also associated with animal farming (Sultana et al (2014). Comparison of water use in global milk production for different typical farms. Agricultural Systems, Volume 129, pages 9-21; Ercin and Aldaya (2012) The water footprint of soy milk and soy burger and equivalent animal products. Ecological Indicators, Volume 18, pages 392-402). In addition, farming effluents also have a strong environmental impact. Although the impact of crop fertilizers on groundwater should not be neglected, the impact of animal farming effluents is often massive and has proven to be disastrous in many parts of the world (https://www.nrdc.org/issues/livestock-production). Furthermore, livestock takes up to nearly 80% of global agricultural land, yet produces less than 20% of the world's supply of calories, showing the very high level of stress brought by animal farming on land resources.

Finally, animal welfare has become an increasingly important concern. The scale-up of meat and dairy production and processing has turned it into an intensive industrial process, which is increasingly perceived as ethically not acceptable.

Therefore, there is a strong need for dairy products substitutes alleviating the issues listed above. Since a large part of milk is transformed into cheese (about 38% in Europe), this need translates for a great part in a need for cheese substitutes.

Plant-based substitutes are a potential alternative to conventional dairy products. However, these products, which are often derived from soy, almond, or coconut milk, can be far from mimicking the taste of dairy products. In addition, they are anyway fundamentally different in terms of composition.

Therefore, there is today a need for dairy substitutes, and in particular, cheese substitutes
(i) free of undesired compounds
(ii) similar to the original product in aspect, texture and taste
(iii) equivalent or superior to the original products in terms of nutrition

Cows' milk typical composition is described in Table 1 and more detailed compositions of cow's and other animals milk, including the listing of the different lipids, proteins, salts, vitamins and other nutrients can be found from a large number of sources (https://en.wikipedia.org/wiki/Milk#Cow's_milk; Haug et al (2007) Bovine milk in human nutrition - a review, Lipids Health Dis.; Volume 6, pages 25; Dominguez-Salasa et al (2019) Contributions of Milk Production to Food and Nutrition Security; Encyclopedia of Food Security and Sustainability, Volume 3, pages 278-291).

**Table 1: Cow's milk composition**

| Type of molecule | mass in 1 liter in grams |
|---|---|
| Water | 902 |
| Lactose | 49 |
| Fat | 39 |
| Caseins | 28 |
| Other proteins | 5 |
| Salts and vitamins | 9 |
| Total | 1032 |

Lipids and carbohydrates (but lactose) can be recovered from plant, calcium from inorganic sources, from animal sources or from plants (such as sea weeds). In the case of proteins, other sources have to be considered for animal proteins differ in amino-acid content from plant proteins. Milk's proteins can also be produced by fermentation, another source of ingredients of a non-animal origin. Furthermore, proteins isolated form milk are also individually used as nutritional supplements and other applications. Therefore, milk proteins made by fermentation, independently of other milk components can also be used for other applications than the making of dairy substitutes.

Production by fermentation is based on the growth of bacteria or fungi producing a compound of interest in a fermenter, usually followed by the recovery and purification of the compound of interest. The making of proteins by fermentation is a process that has been used in the food industry, with one of the first examples being recombinant chymosin, the first artificially produced enzyme to be registered and allowed by the US Food and Drug Administration, and represents today a large part (more 80% in the US today) of the rennet market. (Food Biotechnology in the United States: Science, Regulation, and Issues". U.S. Department of State. Retrieved 2006-08-14). Since then, several studies have described the production of milk's constitutive proteins or homologues by fermentation in various microorganisms (see below) and the assembly of ingredients including fermentation proteins to make milk's substitutes has been described several times (US6270827; US5,942,274; WO2018039632; WO2020223700).

However, the industrial production of milk's protein also requires adapted purification procedures, that not only produce the required proteins at a grade compatible with use in the food industry, with a high level of purity, but are also easily scalable at industrial level, and at costs compatible with exploitation. Ideally, such procedures should be as simple as possible, and avoid the use of non-edible chemicals and of steps that are expensive when conducted at very high scale.

The four casein molecules (alpha-S1-, alpha-S2-, beta-, or kappa-caseins), represent more than 80% of milk proteins, and nearly all of cheese proteins, since the other milk proteins (commonly named "whey proteins") such as betalactoglobulin, alpha-lactalbumin, bovine serum albumin, and immunoglobulins are removed in whey after curdling. The solubility of caseins highly depends on pH, temperature and salt concentration (Post et al. (2012) Effect of temperature and pH on the solubility of caseins: Environmental influences on the dissociation of caseins. J. Dairy Sci. Volume 95 : pages 1603-1616), but in milk, they are not found as soluble proteins, but organized in micelles, resulting in a colloidal structure. Casein micelles are roughly spherical particles ranging from 50 to 600 nm in diameter, with an average diameter of around 200 nm (Kruif, Supra-aggregates of casein micelles as a prelude to coagulation (1998) J Dairy Sci, Volume 81 pages 3019-3028; de Kruif et al. Casein micelles and their internal structure (2012) Advances in Colloid and Interface Science, Volume 171-172, pages 36-52). The evolution of this colloid into a curd is at the basis of cheese production (Gillis JC, Ayerbe A, Le fromage 4ème edition, Lavoisier - Technique Et Documentation 20 avril 2018). It is believed that κ-casein has a key role as a stabilizer of the micelle structure.

Caseins can be isolated from milk as sodium, calcium or potassium caseinates, by neutralization after acid-mediated or rennet-mediated coagulation (Sarode et al. (2016) Methods of Manufacture. In: Caballero, B., Finglas, P., and Toldrá, F. (eds.) The Encyclopedia of Food and Health vol. 1, pp. 676-682. Oxford: Academic Press) The different individual caseins (alpha-S1-, alpha-S2-, beta-, or kappa-caseins) can also be purified individually from bulk casein preparations, based on their different physico-chemical properties, using membrane filtration at low temperature (Murphy and Fox (1991) Fractionation of sodium caseinate by ultrafiltration. Food Chem. Volume 39 Pages 27-38:; Ward and Bastian (1996) A method for isolating β-casein. J. Dairy Sci. Volume 79, pages 1332-1339; Huppertz et al. (2006). A method for the large-scale isolation of β-casein. Food Chem. Volume 99, pages 45-50; Lamotheet al. (2007). Short communication: Extraction of β-casein from goat milk. J. Dairy Sci. Volume 90, pages 5380-5382; O'Mahony, et al. (2007). Purification of β-casein from milk. US Pat. No. 0104847 A1) or selective precipitation (Law and Leaver (2007) Methods of extracting casein fractions from milk and caseinates and production of novel products. Hanna Research Institute, assignee. WO Pat. No. 03003847; Post et al. (2009). β-Casein as a bioactive precursor-Processing for purification. Aust. J. Dairy Technol. Volume 64, pages 84-88; Post and Hinrichs (2011) Large-scale isolation of food-grade β-casein. Milchwissenschaft, Volume 66, pages 361-364).

In the case of recombinant proteins made by fermentation, specific procedures have to be developed, in order to isolate the recombinant protein from the biomass and from the culture broth, and such procedures depend on the production microbe and on the properties of the recombinant protein.

In *Escherichia coli,* one of the workhorses of protein production, recombinant proteins are often found in inclusion bodies. These intracellular particles consist essentially in aggregates of the recombinant protein. Recovering properly folded proteins from such aggregates requires a tedious refolding process (Singh et al. (2015) Protein recovery from inclusion bodies of Escherichia coli using mild solubilization process. Microbial Cell Factories, Volume 14: pages 41-51; Vallejo and Rinas (2004) Strategies for the recovery of active proteins through refolding of bacterial inclusion body proteins. Microbial Cell Factories Volume 3: pages 11). However, inclusion bodies are small particles (0.2-1.5 mm) and protocols for their isolation are often based on high relative centrifugal force centrifugation (Rodriguez-Carmona et al. (2010) Isolation of cell-free bacterial inclusion bodies. Microbial Cell Factories Volume 9, pages 71), a step that is not easily brought at industrial scale, which can be problematic for large size batches.

For thermoresistant recombinant proteins or peptides, using thermal lysis can be an interesting purification step, for a lot of the host soluble proteins will precipitate and/or be degraded at high temperature (Takesawa et al. (1990) Heat-induced precipitation of cell homogenates: an investigation of the recovery of thermostable proteins. Enzyme Microb. Technol. Volume 12, pages 184-189; Kirk and Cowan 1995 Optimising the recovery of recombinant thermostable proteins expressed in mesophilic hosts. J. of Biotechnology Volume 42, pages 177-184; Sundarrajan et al. (2018) Novel properties of recombinant Sso7d-Taq DNA polymerase purified using aqueous two-phase extraction: Utilities of the enzyme in viral diagnosis. Biotechnol Rep (Amst)19:e00270; US8603782; WO2011119703).

Post et al (Journal of Dairy Science, vol. 95, no. 4, 2012, 1603-1616) are not concerned with casein purification, but analyze the reasons for the existence of selective precipitation of beta casein over alpha casein. The information provided in this document is thus not related with the technical problem of isolating casein, especially recombinant caseins.

US 4 550 028 does not describe the use of recombinant caseins, nor their preparation or purification. One can note that this document (column 3, lines 1-22) refers to the flocculation of casein at an acidic pH with an increased temperature, which is different from the technical effect herein achieved (where casein remains in solution, while the other proteins, which are not milk proteins, precipitate).

WO 99/54355 describes the purification and recovery of a recombinant protein from a cell using a water-miscible organic solvent (in particular acetone, isopropanol, ethanol and methanol) at a pH of about 5.0 to about 10.0 and at a temperature of about 30°C to about 50°C. The application states (page 6, lines 20-24) that *"The water-miscible organic solvent extracts the recombinant protein from the suspension of cells. The water-miscible organic solvent is believed to selectively partition the recombinant protein from the suspension of cells and into a liquid phase".* Even though the purpose is similar to the one of the present application (purification of recombinant protein), the technical effect is obtained by another method (use of an organic solvent) than herein disclosed.

Indeed, it is important, for food application, to avoid the use of chemicals such as organic solvents, which will have to be removed in subsequent steps, and the processes herein disclosed are performed on the bacterial culture medium (preferably after lysis of the cells and removal of the cells debris) which is devoid of organic solvents.

WO 2020/223700 relates to the preparation of recombinant casein-based formulas (alpha and kappa casein in micellar form, preferably without beta casein as indicated in [0006]). This document does not describe the purification of these recombinant proteins, nor their use, and one can also note that the examples relate to the manufacture of fresh products being made from micellar casein *"typically obtained in industry by ultrafiltration of skim milk to isolate casein micelles and spray drying techniques to powderize casein micelles"* as seen in [00210] or after reconstitution of micelles (Example 6).

US 4,519,945 relates to the preparation of a curd from milk, by adjusting the pH and temperature. This document thus relates to conditions for obtaining flocculation of caseins, and is not related to purpose of isolating and purifying caseins.

The Applicant has shown that caseins become or remain solubilized at a high temperature, whereas other proteins precipitate and/or are being degraded. In particular, the Applicant has found that in *E. coli,* recombinant caseins (and in particular alpha and beta caseins), which are found in the insoluble fraction, are solubilized by heating at high temperature (higher than 75°C), while in contrast, many soluble proteins from the host were precipitated and/or degraded at such temperatures. Using such property, it is possible to eliminate all or part of non-casein proteins in a composition, and thus enrich in (i.e. increase the relative amount of) casein in the composition. A process to isolate caseins from bacterial cells was thus developed and is the basis of the invention herein disclosed.

The invention is defined by the claims.

In the context of the present invention, a "casein" is any casein protein or mixture of casein protein. Thus, a "casein" is an alpha-S1 casein, an alpha-S2 casein, a beta casein, or a kappa casein. In some extend, it can also designate any mixture of such proteins. One can use the term "casein" or "caseins" to discuss casein proteins in general.

In the context of the invention, the term "between" includes the limits.

In the context of the present invention, the term "cheese substitute" means a food product having the essential features of cheese in terms of nutritional value, aspect, texture and taste.

The term "fresh cheese" designates a cheese with a moisture higher than 80% on a fat-free basis (ratio of water vs. total mass of product without fat), and a protein content comprised between 2% and 15% of total weight (ratio of protein mass vs. total product mass).

The term "soft cheese" or "semi-soft cheese" designates a cheese with a moisture comprised between 62% and 80% on a fat-free basis (ratio of water vs. total mass of product without fat), and a protein content comprised between 15% and 30% of total weight (ratio of protein mass vs. total product mass). A soft cheese has a moisture comprised between 67% and 80%, on a fat-free basis, and a semi-soft cheese has a moisture comprised between 62% and 67%, on a fat-free basis.

In the context of the present invention, the term "liquid pre-curd composition" or "LpCC" designates the composition containing at least a casein and at least one other ingredient comprising at least a component among water, calcium, lipids, an carbohydrate, before the addition of rennet and ferments and curdling (in particular coagulation of casein). The casein concentration in the LpCC is higher than that in milk. The concentration of the other ingredient in the LpCC is also higher than that in milk. This makes it possible to save some water when using a LpCC with recombinant caseins as disclosed in the present application, as the LpCC doesn't represent an artificial (cow's) milk. The respective concentrations of proteins, lipids, calcium salts and/or carbohydrates are adjusted to fit the final desired compositions of cheese substitutes

In the context of the present invention, the term "curdling agent" designates a chemical or biochemical composition capable of triggering curdling. Curdling can be achieved by the addition of acid solutions, by the addition of starter cultures (ferments which growth in the presence of carbohydrates triggers a decrease of pH), by heat treatment, by addition of calcium chelating agent, by the addition of natural or recombinant rennet, by the addition of rennet substitutes, such as animal proteases, or vegetal curdling enzymes), or by a combination of these processes. A curdling agent may be any additive, compound, composition or treatment which addition results in curdling, alone or in combination with another or others additives, compounds, compositions or treatments. When preparing animal-free edible compositions, it is preferred to use an acidifying agent (such as an acid or starter cultures), in order to avoid using rennet, so that the curdling agent does not comprise any element of animal origin. It is advantageous when the acidifying agent comprises a lactic bacterium or lactic bacteria, but acidic chemicals can also be used.

In the context of the present invention, the term "curd" means a composition wherein casein coagulates or precipitate, under the action of the curdling agent, and which can be separated from a liquid phase, if any, by draining, for example on a cheese cloth. The curd also comprises other ingredients, including water and lipids (when present),

In the context of the present invention, the term "ferment" designates a composition containing at least one microbial strain, added during the process of production of the cheese substitute. Lactic ferments are responsible for lactic fermentation, resulting notably in an acidification of the medium. As a consequence, they can be used as a curdling agent.

Other ferments are used in cheese production for the processing of the curd, resulting in modifications of texture, taste, smell, and chemical composition (with notably the cleavage of proteins into smaller peptides). These ferments can be called "ferments for maturation", "maturation ferments" or "ripening ferments" and are generally not used for production of fresh cheeses. Such ferments for maturation can be added together with the curdling agent.

In the context of the present invention, the term "of non-animal origin" means a compound or composition which has not been directly derived from an animal, produced from animal cells in culture, or isolated from animal products such as milk. Compounds or compositions produced by fermentation of microbes are thus "of non-animal origin" even though some products of animal origin, bacto peptone for example, can be involved during fermentation. Therefore, in the context of the invention, a protein that is naturally produced in animals will be called of non-animal origin when it is produced in microbial (such as bacterial or yeast) cells or in plant cells, even though its sequence or structure may be identical to the sequence or structure of the protein that would be isolated from animal.

In the context of the present invention, the term "animal-free" means a compound or composition which has not been derived from an animal, from animal cells in culture, or from animal products such as milk, and whose production process does not involve any feedstock or additive of animal origin.

In the context of the invention, the term "texturing agent" means any gelling agent, including emulsifier such as lecithin, and hydrocolloids such as cassia gum, sesbania gum, tamarind gum, guar gum, fenugreek gum, Arabic gum, agar agar (or agar-agar), carrageenans, tragacanth gum, xanthan gum, carob (locust bean) gum, cellulose gum.

In a first aspect, is herein disclosed a method for the production of a composition containing casein composition, comprising:
i) providing a casein composition and other proteins wherein the pH of the composition is equal or above 6.5 and preferably below 9
ii) heating the composition so as to reduce (or diminish) the amount of the other proteins in the soluble fraction of the composition,
iii) recovering the soluble fraction
thereby obtaining a composition of casein.

In particular, in ii), heating increases the ratio of casein versus other proteins in the soluble fraction, and thus enriches the composition in casein.

The composition of i) contains a soluble fraction and optionally an insoluble fraction. The soluble fraction refers to a fraction that is not pelleted upon centrifugation. The insoluble fraction is the pellet obtained after centrifugation. Centrifugation is preferably performed at about 3000 g for 20-30 minutes. At industrial scale, continuous flow centrifugation, or another method (such as filtration) may be used to recover the soluble fraction.

The desired technical effect is obtained by heating, and preferably in the absence of organic solvents, or of addition thereof.

The ratio of casein is increased in the soluble fraction, when the relative amount of casein increases as compared to the total amount of proteins.

Such method can be used for the isolation or purification of casein from the composition containing casein and other proteins. Such method can also be used for enriching casein in a protein composition, *i.e.* increasing the proportion of casein in the protein fraction of the protein composition.

In the context of the methods herein described, a "casein composition" designates a composition which comprises microorganisms that have been transformed with at least one nucleic acid coding for a casein, and the microorganisms have been cultured so as to express and produce casein, wherein the microorganisms also express other proteins. In order to use a purification step based on the separation of the soluble and insoluble fraction, a casein composition needs to comprise a soluble fraction. However, this casein composition can then be desiccated in further steps. In some embodiments, casein represents more than 25 % of the proteins of the composition. In another embodiment casein represents more than 50 % of the proteins of the composition. In come embodiments, casein represents the major part (more than 50%) of dry weight. Such casein composition can also comprise other compounds, notably calcium, other proteins, lipids and others. A casein composition produced by a microbial culture is a casein composition of non-animal origin.

One can also cite a method for the enrichment of casein in a casein composition containing casein and other proteins, comprising:
i) providing a casein composition containing casein and other proteins
ii) heating the composition so as reduce the amount of the other proteins in the soluble fraction of the composition, while increasing or maintaining the amount of casein in the soluble fraction, and
iii) recovering the soluble fraction
thereby enriching casein in the composition.

The other proteins are non-casein proteins. This method thus makes it possible to obtain a composition enriched in casein. Enrichment of casein in the composition refers to an increase of casein ratio (or relative amount) in the whole protein content. It is compared to the amount before heating.

The desired technical effect is obtained by heating, and preferably in the absence of organic solvents or of addition thereof.

In view of this, it results that the casein composition may contain only casein at the end of the process, or may also contain other proteins (when the precipitation and/or degradation of the other proteins upon heating is not complete). However, as indicated above, it is preferred when casein represents at least 50% of the proteins in the composition (weight/weight) obtained after performing the process. In a preferred embodiment, casein represents at least 60 %, or at least 70% or at least 80%, or at least 85%, or at least 90%,or at least 91%, or at least 92%, or at least 93%, or at least 94%, or at least 95% of the proteins of the composition obtained after performing the process. The amount of proteins can be measured by any method known in the art (using a gel, protein dosing).

As indicated above, the composition containing casein and other proteins, which is used at (i) may also contain other components, such as carbohydrate or lipids. This is particularly true when the composition containing casein and other proteins results from a culture of recombinant microorganisms (including eukaryotic cells) expressing casein. Such other components (in totality or part of such) will be present in the casein composition.

Such composition containing casein and other proteins used in (i) may contain the microorganisms suspended in an appropriate buffer or solution but more preferably in water, or the supernatant resulting from the microorganisms' culture, in case the casein is secreted by the microorganisms.

In a specific embodiment, the starting composition (composition containing casein and other proteins) contains alpha-S1 casein. In one embodiment, alpha-S1 casein is the only casein in the starting composition.

In a specific embodiment, the starting composition (composition containing casein and other proteins) contains alpha-S2 casein. In one embodiment, alpha-S2 casein is the only casein in the starting composition.

In a specific embodiment, the starting composition (composition containing casein and other proteins) contains beta casein. In one embodiment, beta casein is the only casein in the starting composition.

In a specific, but non-preferred embodiment, the starting composition (composition containing casein and other proteins) contains kappa casein. Thus in preferred embodiments, the starting composition (composition containing casein and other proteins) doesn't contain kappa casein.

In a specific embodiment, the starting composition (composition containing casein and other proteins) contains a mixture of alpha-S1 and beta caseins. In one embodiment, alpha-S1 and beta caseins are the only caseins in the starting composition.

In a specific embodiment, the starting composition (composition containing casein and other proteins) contains a mixture of alpha-S1 and alpha-S2 caseins. In one embodiment, alpha-S1 and alpha-S2 caseins are the only caseins in the starting composition.

In a specific embodiment, the starting composition (composition containing casein and other proteins) contains a mixture of alpha-S2 and beta caseins. In one embodiment, alpha-S2 and beta caseins are the only caseins in the starting composition.

In a specific embodiment, the starting composition (composition containing casein and other proteins) contains a mixture of alpha-S1, alpha-S2 and beta caseins. In one embodiment, alpha-S1, alpha-S2 and beta caseins are the only caseins in the starting composition.

In a specific embodiment, the starting composition (composition containing casein and other proteins) contains a mixture of alpha-S1, alpha-S2, beta and kappa caseins.

In a specific embodiment, the starting composition (composition containing casein and other proteins) contains a mixture of a combination of two to three caseins chosen among alpha-S1, alpha-S2, beta and kappa caseins.

The soluble fraction may be subjected to another step of purification, such as filtration (in particular ultrafiltration, nanofiltration, reverse osmosis), chromatography, or another precipitation, such as acidic precipitation of casein) to further purify and/or enrich the casein. In particular, acidic precipitation is performed at pH=4, and preferably at about 90°C. Multiple other steps of purification can be performed. One can also add activated charcoal in the soluble fraction, before or after operating one or more of the above purification methods.

In a specific embodiment, the microorganisms are bacterial cells. In another embodiment, the microorganisms are fungi cells (including yeast cells). In another embodiment, the microorganisms are eukaryotic cells, in particular plant cells. It is indeed preferred that the casein is produced in a non-animal organism.

As the casein is produced by way of culture or fermentation of micoorganisms (in particular bacteria) transformed to express casein, one can use the properties of the casein (remain soluble at high temperature, whereas other proteins precipitate and/or are being degraded) to perform a method for the production of a composition of casein, comprising:
i) providing a microorganism composition (preferably bacterial composition), wherein the microorganism composition (preferably bacterial composition) comprises microorganisms (preferably bacteria) that have been transformed with at least one nucleic acid coding for a casein, and the microorganisms (preferably bacteria) have been cultured so as to express and produce casein
ii) heating the microorganism composition (preferably bacterial composition) at a temperature inducing enrichment of casein in the soluble fraction and/or reduction of the amount of the other proteins in soluble fraction
iii) isolating the soluble fraction from the heated solution of ii),
iv) optionally performing one further step chosen among addition of activated charcoal, membrane filtration (ultrafiltration, nanofiltration, or reverse osmosis ), chromatography or precipitation of casein applied to the soluble fraction isolated in iii),
thereby obtaining a composition of casein.

In ii), heating the microorganism composition (preferably bacterial composition) shall also induce lysis of the cells, if they are not already lysed, so as to produce a microbial extract, wherein the soluble fraction is enriched in casein and/or wherein the amount of other proteins is reduced in the soluble fraction.

Thus, heating in ii) will enrich in casein in the soluble fraction and/or reduce the amount of the other proteins in soluble fraction, when the microorganism composition comprises already lysed microorganisms, and a further effect of lysis of the microorganisms is observed when the microorganisms present in the microorganism composition are not already lysed.

The enrichment in casein or reduction of other proteins in the soluble fraction is observed as compared to the soluble fraction of the microorganism composition in which the microorganisms have been lysed before heating.

Such method thus allows obtaining a composition of casein, and isolation of a casein-rich (or casein-enriched) composition from a microorganism culture. A casein-rich composition is a composition wherein casein represent the major protein. In a preferred embodiment, caseins represent more than 50% of proteins in said casein-rich composition. In a more preferred embodiment, casein represent more than 80%, or 90% or 95% of proteins in said casein-rich composition.

Indeed, and as shown in the examples, upon heating, caseins move from the insoluble fraction (pellet) to the soluble fraction (supernatant). The examples also show that it is essentially casein that is transferred to the soluble fraction upon action of heat.

The microorganism composition contains microorganism cells suspended in an appropriate fluid (such as water or other buffer as explained below). Therefore, it is possible, therefore, upon centrifugation, to obtain an insoluble (pellet) fraction and a soluble fraction (supernatant). It is preferred, however, when the method is performed before centrifugation. In this embodiment, the insoluble fraction can be in suspension within the soluble fraction when heating is performed. Centrifugation, or another method (such as filtration) may thus be used to recover the soluble fraction.

In one embodiment, the microorganism composition (preferably bacterial composition) has been obtained by centrifugation of the microorganism cells (preferably bacteria) after culture, washing, and resuspension in an appropriate liquid or fluid (appropriate buffer and more preferably in water, more preferably in the absence of organic solvents). The buffer is at a pH that is not acidic (equal or above 6.5), preferably close to neutral (between 6.5 and 7.5), or basic (preferably below 9). Indeed, caseins can precipitate at acidic pH, and the process is even enhanced by heating as shown in example 7. It is also preferred when the buffer is not ionic, or with a low ionic strength. The soluble fraction of iii) can be obtained by centrifugation of the composition heated in ii), or by other methods known in the art.

The composition of i) in the methods disclosed above is heated, in ii), at a temperature comprised between 75°C and 105°C, in particular between about 75°C and about 100°C. Such temperatures make it possible to obtain the technical and functional effects recited in ii) above (removal of the other proteins, therefore reducing their amount and/or moving the casein from the insoluble to the soluble fraction). It is possible to work at temperatures above 100°C, even when water is used if the pressure is increased. One of skill in the art is able to determine the proper conditions of temperature, pressure and pH to perform the methods herein disclosed.

The term "about", when referring to a measurable value, is meant to encompass variations of ±4% from the specified value, as such variations are appropriate to perform the disclosed methods.

In particular, the temperature is equal or above 80°C, or equal or above 85°C, equal or above 90°C. In particular, it is comprised between about 85°C and about 95°C, more specifically is about 95°C. In another embodiment, the temperature is comprised between about 90°C and about 95°C. The temperature is generally equal or below 100°C. Temperatures between 80°C and 100°C or 85°C and 100°C are suitable.

The duration of the heating can be adjusted by the person skilled in the art. When heating is performed at about 95°C, the duration may be between about 5 minutes and about 2 hours. It is preferred when the duration is at least 10 minutes, 20 minutes, 30 minutes, 40 minutes, one hour, more preferably about 2 hours. It can be extended (in particular for lower temperatures, or shortened, in particular for higher temperatures).

As indicated above, a specific embodiment encompasses the situation when the starting composition (composition containing the casein and other proteins) has been obtained from a bacterial culture. In such embodiment, the bacteria have been transformed with one or more nucleic acid coding for more than one casein.

In particular, in one embodiment, the bacteria have been transformed with one or more nucleic acid coding for a beta casein.

In particular, in one embodiment, the bacteria have been transformed with one or more nucleic acid coding for an alpha-S1 casein.

In particular, in one embodiment, the bacteria have been transformed with one or more nucleic acid coding for an alpha-S2 casein.

In particular, in one embodiment, the bacteria have been transformed with one or more nucleic acid coding for both a beta and an alpha-S1 casein.

In particular, in one embodiment, the bacteria have been transformed with one or more nucleic acid coding for a beta an alpha-S1 casein and an alpha-S2 casein, or a combination of two out of them.

It is to be noted that, when multiple proteins are produced, the bacteria can be transformed with different nucleic acids (each one coding for a different protein) or with a unique nucleic acid (which contains the elements allowing production of the various proteins). Such methods of transforming microorganisms and bacteria in particular, for production of one or of multiple proteins are known in the art.

The nucleic acids are such that they contain all elements allowing production of the casein that they encode (such as promoters, enhancers, terminators and the like). The person skilled in the art knows all these elements and is able to select the ones that are the most appropriate. The person skilled in the art can also select the microorganisms to use to produce the caseins.

In particular, prokaryotic hosts suitable for expressing caseins include *E. coli, Bacillus subtilis, Salmonella typhimurium, Lactococcus lactis* and various species within the genera *Lactococcus, Pseudomonas, Streptomyces,* and *Staphylococcus.*

Eukaryotic host suitable for expressing caseins include fungi, such as *Saccharomyces cerevisae, Kluyveromyces lactis, Pichia pastoris, or Trichoderma reesei*)*.*

Plant cells may also be used for producing protein, either in cell culture or in whole plants.

Further steps can be added such as addition of activated charcoal, membrane filtration, chromatography or precipitation of casein, resulting in a process comprising
i) providing a microorganism composition (preferably bacterial composition), wherein the microorganism composition (preferably bacterial composition) comprises microorganisms (preferably bacteria) that have been transformed with at least one nucleic acid coding for a casein, and the microorganisms (preferably bacteria) have been cultured so as to express and produce casein
ii) heating the microorganism composition (preferably bacterial composition) at a temperature inducing enrichment of casein in the soluble fraction and/or reduction of the amount of other proteins in the soluble fraction
iii) isolating the soluble fraction from the heated composition of ii),
iv) further processing said soluble fraction by adding at least one step chosen among addition of activated charcoal, membrane filtration, chromatography or casein precipitation
thereby obtaining a casein composition.

In this embodiment, heating in ii) also induces lysis of the microorganisms, so that a microbial extract is obtained, and casein is enriched and/or other proteins are reduced in this extract.

In addition, the heating process can be used independently of cell lysis, in a process comprising:
i) providing a microbial extract, wherein the microbial extract results from the lysis of a microbial culture comprising micro-organisms that have been transformed with a nucleic acid coding for the casein, and cultured so as to express and produce casein
ii) heating the microbial extract at a temperature so as to enrich casein in the soluble fraction and/or reduce the amount of the other proteins in soluble fraction of the composition
iii) isolating the soluble fraction after heating
iv) further processing said soluble fraction by adding at least one step chosen among addition of activated charcoal, membrane filtration, chromatography or casein precipitation
thereby obtaining a casein composition.

The soluble fraction isolated in iii) can be further processed for increasing purity of casein.

Activated charcoal can be added to the soluble fraction and stirring is performed. Activated charcoal can be used to adsorb impurities (in particular organic impurities or chlorine) present in the soluble fraction, and that were not removed when recovering the soluble fraction in iii). Stirring with activated charcoal is preferably done, and the activated charcoal is removed before another method (such as the ones disclosed below) is performed on the soluble fraction.

Chromatography methods are widely used for the purification methods, and include notably affinity chromatography, ion exchange chromatography, hydrophobic interaction chromatography and others.

Membrane filtration, including notably ultrafiltration and nanofiltration is commonly used in protein purification as well (Saxena et al. (2009) Membrane-based techniques for the separation and purification of proteins: An overview. Advances in colloids and Interface Science Volume 145, pages 1-22), and membrane filtration techniques are widely used in the dairy industry. Interestingly, they can be used to separate the different caseins from each other (see above).

Finally, the specific properties of caseins (Post et al. (2012) Effect of temperature and pH on the solubility of caseins: Environmental influences on the dissociation of caseins. J. Dairy Sci. Volume 95 : pages 1603-1616), and notably, their propensity to precipitate in acidic conditions can be used for further purification.

In a preferred embodiment, further processing said soluble fraction is done by precipitation of caseins in acidic conditions. After precipitation, caseins can be resuspended, and using an appropriate basic buffer, they can also be resolubilized (Post et al. (2012) Effect of temperature and pH on the solubility of caseins: Environmental influences on the dissociation of caseins. J. Dairy Sci. Volume 95 : pages 1603-1616) Precipitation in acidic conditions is particularly interesting when the casein is isolated from a culture of microorganisms, as it makes it possible to remove or degrade any nucleic acid that may be present in the casein composition. Presence of DNA or RNA of the microorganisms may prove to be detrimental (at least from a regulatory point of view) when the casein is to be used for obtaining edible compositions intended to be ingested by human beings (such as cheese substitutes).

In a specific embodiment, the casein composition is desiccated. When obtained from a bacterial culture, such composition shall contain about 15-30% casein (w/w) and carbohydrates in the desiccated composition.

It is preferred when a little water is left, to favor future rehydration. Consequently, desiccation is to be understood as reducing the amount of water. In some embodiments, the amount of water is about 50% or less (w/w).

Is also disclosed a method for the production of a composition of casein comprising:
i) providing a bacterial composition, wherein the bacterial composition comprises bacteria that have been transformed with at least one nucleic acid coding for a casein, and the bacteria have been cultured so as to express and produce casein,
ii) heating the bacterial composition at a temperature inducing enrichment of casein in the soluble fraction and/or reduction of the amount of the other proteins in the soluble fraction ,
iii) isolating the soluble fraction from the heated cell composition of ii),
iv) further processing said soluble fraction by adding at least one step chosen among addition of activated charcoal, membrane filtration, chromatography or casein precipitation
thereby obtaining a composition of casein.

When the bacteria of the bacterial composition are not lysed, heating in ii) also induces lysis of the cells, which produces a bacterial extract.

Is also disclosed a method for the production of a composition of casein comprising:
i) providing a bacterial composition, wherein the bacterial composition comprises bacteria that have been transformed with at least one nucleic acid coding for a casein, and the bacteria have been cultured so as to express and produce casein,
ii) heating the bacterial composition at a temperature inducing enrichment of casein in the soluble fraction and/or reduction of the amount of other proteins in the soluble fraction,
iii) isolating the soluble fraction from the heated bacterial composition of ii),
iv) further processing said soluble fraction by precipitation of casein in acidic conditions
thereby obtaining a composition of casein.

In this embodiment, when the bacteria of the bacterial composition are not lysed, heating in ii) shall also induce lysis of the bacteria, thereby producing a bacterial extract.

Is also disclosed a method for the production of a composition of casein comprising:
i) providing a bacterial composition, wherein the bacterial composition comprises bacteria that have been transformed with at least one nucleic acid coding for a casein, and the bacteria have been cultured so as to express and produce casein,
ii) heating the bacterial composition at a temperature inducing enrichment of casein in the soluble fraction and/or reduction of the amount of other proteins in the soluble fraction,
iii) isolating the soluble fraction from the heated bacterial composition of ii),
iv) further processing said soluble fraction by precipitation of casein by heating at 90°C at pH=4
thereby obtaining a composition of casein.

In a preferred embodiment, heating the bacterial composition induces lysis of the cells.

The methods herein disclosed thus make it possible to obtain a composition of casein. Such composition of casein susceptible to be obtained (or obtained) by a method herein disclosed can be characterized as defined above.

In milk, the casein is in the micellar form found in milk, wherein micelles include alpha-S1, alpha-S2, beta and kappa caseins assembled in the same particules. In the context of the present description, casein may not be assembled in the micellar form. However, and as shown below, this doesn't prevent ability to obtain a curd from the composition of casein, by addition of an appropriate curdling agent.

Indeed, as developed in the examples, such composition of casein can be used for producing cheese substitutes, and in particular animal-free cheese substitutes, *i.e.* cheese substitutes that don't contain any products of animal origin (in particular when the casein has been isolated from bacterial or yeast culture).

In summary, simple heating of a composition containing casein and other proteins makes it possible to enrich the composition in casein by reducing the amount of most of the other proteins in the soluble fraction. Heating also makes it possible to solubilize casein found in the insoluble fraction of bacterial cultures. The obtained casein, although not in the optimal condition for forming micelles, can appropriately curd upon the action of a curdling agent. Such findings were unexpected.

In particular, it was shown that curdling can be achieved with the recombinant caseins made in bacteria, and in the absence of kappa caseins.

It is known that dairy caseins, notably alpha-S1, alpha-S2 and beta caseins, are phosphorylated; in addition, kappa caseins are glycosylated (Walstra et al. (2006) Dairy Science and Technology. Taylor and Francis Group, Boca Ranton, USA; Martin et al., (2003) Non bovine caseins quantitative variability and molecular diversity. In: Fox PF and Mc Sweeney PLH. Advances in Dairy Chemistry - Proteins, Vol1, Springer, New-York, 227-310). Caseins phosphorylations play an important role in the interactions with calcium and in the structure of dairy casein micelles. Such post-translational modifications will not be found in recombinant caseins made in bacteria.

It is also known that kappa caseins also play an important role in the formation of dairy casein micelles. WO2020223700 described the making of curds with recombinant alpha-S1 casein (dephosphorylated) and kappa casein (deglycosylated), after the formation of micelles formed with these two caseins (see example 14 of this document). Yet, WO2020223700 also reports that the use of hypophosphorylated alpha casein results in looser micelles (see example 11 of this document). The fact that curdling can be achieved in the absence of post-translational modifications and of kappa caseins is thus unexpected.

The compositions herein disclosed, and obtained or susceptible to be obtained by the methods disclosed above, thus can be used in a method for obtaining a curd, comprising
i) providing a composition of casein herein disclosed,
ii) mixing said composition of casein with at least one other ingredient comprising at least one component selected from the group consisting of water, calcium, lipids, and carbohydrate, so as to obtain a liquid pre-curd composition (LpCC)
iii) Adding at least one curdling agent to the liquid composition so as to obtain a curd.
In particular, said curdling agent is not rennet, but is an acidifying agent. In a preferred embodiment, said curdling agent is a lactic bacteria, or lactic, citric or acetic acid. In a preferred embodiment, the casein composition doesn't contain any kappa casein. In a preferred embodiment, the caseins don't contain any post-translational modification. In particular, the composition of casein only contains alpha-S1 casein (as the casein protein). In particular, the composition of casein only contains alpha-S2 casein (as the casein protein). In particular, the composition of casein only contains beta casein (as the casein protein). In particular, the composition of casein only contains alpha-S1 and beta casein (as the casein proteins). In one embodiment casein composition only contains alpha-S2 and beta casein (as the casein proteins). In particular, the composition of casein only contains alpha-S1 and alpha-S2 casein (as the casein proteins). In one embodiment casein composition contains alpha-S1, alpha-S2 and beta casein.

One can thus perform a method for producing an edible composition, comprising
i) providing a composition of casein herein disclosed,
ii) mixing said composition of casein with at least one other ingredient comprising at least one component selected from the group consisting of water, calcium, lipids, and carbohydrate, so as to obtain a liquid pre-curd composition (LpCC)
iii) Adding at least one curdling agent to the liquid composition so as to obtain a curd, and
iv) Further processing the curd to obtain an edible composition
In a preferred embodiment, said curdling agent is not rennet, but is an acidifying agent. In a preferred embodiment, said curdling agent is a lactic bacteria or lactic, citric or acetic acid.

In the above embodiments, it is preferred when a gelling agent is added in b) or in d).

It is preferred when the other ingredients added in b) are all of non-animal origin. In the methods for producing curds and edible compositions herein disclosed, it is preferred to use only products of non-animal origin so as to obtain edible composition containing no element of animal origin.

In preferred embodiments, the ingredients added in b) comprise
i. Optionally proteins other than caseins
ii. Lipids
iii. Water, and
iv. Carbohydrates; in preferred embodiments, the carbohydrates do not comprise lactose, in order for the edible composition to be more acceptable to lactose-intolerant customers.

Generally, edible composition
i. has a protein content comprised of less than 25%, preferably between 2 and 25 % or between 5 and 25 % (w/w)
ii. has a moisture content higher than 62%, on a fat-free basis.

In the preferred embodiment, the edible composition is a cheese substitute.

In particular, the composition of casein contains at least one casein among alpha-S1-, alpha-S2-, beta-, or kappa-caseins. In particular, the composition of casein contains at least two caseins among alpha-S1-, alpha-S2-, beta-, or kappa-caseins. In particular, the composition of the casein contains alpha-S1-, alpha-S2-, beta-, or kappa-caseins. In an embodiment, at least one casein selected in the group consisting of alpha-S1 casein, alpha-S2 casein, beta casein and kappa casein is not present in the casein composition. In an embodiment, kappa casein is not present in the composition of casein. In particular, the composition of casein contains only beta casein. In particular, the composition of casein contains only beta and alpha-S2 caseins. In particular, the composition of casein contains only beta and alpha-S1 caseins. In particular, the composition of casein contains only beta, alpha-S1 and alpha-S2 caseins. Indeed, the inventors have shown that it is possible to obtain a curd, and an edible composition that is a cheese substitute, without kappa casein, so that the inventors did not try to reconstitute micelle formation, or check whether the solution actually presents micelles.

When preparing animal-free edible compositions, it is preferred to use an acidifying agent (such as an acid or starter cultures), in order to avoid using rennet, so that the curdling agent does not comprise any element of animal origin. It is advantageous when the acidifying agent comprises a lactic bacterium or lactic bacteria.

The composition of the LpCC is adapted to the eventual composition desired for the edible composition, taking into account the loss of water due to curdling and aging (which can be modulated by one of skill in the art by modifying the duration and conditions of maturation).

In one embodiment, the cheese substitute has the essential features of fresh cheese. The fresh cheese contains a moisture of 80% or higher, on a fat-free basis. The percentage of moisture on a fat-free basis is the mass of water present in the product, divided by the total mass minus the mass of fat (=mass water/total mass - mass of fat). In fresh cheese, protein content is usually lower than 15%, and can be as low as 7% of total weight or even lower.

In this embodiment, the edible composition has preferably
i. a protein content comprised between 2 and 15% (weight content)
ii. a moisture content superior to 80%, on a fat-free basis

More specifically, the edible composition would have a protein content comprised between 5 and 15% (weight content).

It is possible to obtain these characteristics when the the LpCC obtain in b)
i. has a protein content inferior to 12% (weight content)
ii. has a moisture content superior to 80%, on a fat-free basis.

In particular, the LpCC obtained in b)
i. has a protein content comprised between 5% and 10%% (weight content)
ii. has a moisture content superior to 80%, on a fat-free basis.

In this embodiment, it is preferred when agar-agar is added to the LpCC in an amount to represent 0,5% to 0,8% (w/w) of LpCC.

Some other characteristics of the edible composition or of the LpCC used during the process for making the edible composition are described below, and can be found alone or in composition:
- the edible composition has a lipid content comprised between 10% and 30% (weight content)
- the LpCC has a lipid content comprised between 10% and 30% (weight content)
- the edible composition has a calcium content lower than 2.8 % (weight content)
- the LpCC has a calcium content comprised between 0.1% to 1% (weight content)

When performing the method to obtain an edible composition that would be a fresh cheese substitute, processing of the curd may include the steps of
a) mixing a structuring agent with the curd, wherein the structuring agent is selected in the group consisting of gelling agents, texturing agents, emulsifying agents and mixtures thereof, to obtain a supplemented curd, and
b) molding and draining the supplemented curd.
c) Other embodiments are also disclosed below.

In this embodiment, is disclosed a method for the production of an edible composition comprising:
a) Providing a composition of casein, comprising recombinant casein as herein disclosed (preferably only recombinant casein as herein disclosed, preferably only casein of non-animal origin)
b) Mixing said composition of casein with at least one other ingredient comprising at least one component selected from the group consisting of water, calcium, lipids, and carbohydrate, so as to obtain a liquid pre-curd composition (LpCC),
c) Adding at least one curdling agent to the liquid pre-curd composition so as to obtain a curd
d) Further processing the curd to obtain an edible composition,
wherein a gelling agent is added in b) and/or in d).

"Comprising only casein of non-animal origin" indicates that no casein of animal origin is present (i.e. comprising casein that is only of non-animal origin). In preferred embodiments, if other elements are present, they also are of non-animal origin. In other embodiments, the casein composition doesn't contain any other elements than casein (of non-animal origin, preferably the recombinant casein herein described).

In one embodiment, the gelling agent is added in b).

In another embodiment, the gelling agent is added in d).

In another embodiment, the gelling agent is added in b) and in d).

In this embodiment, it is preferred when the edible composition
i. has a protein content comprised between 2 and 15% (weight content)
ii. has a moisture content superior to 80%, on a fat-free basis.

This embodiment is thus well adapted for the production of a fresh cheese substitute.

In another preferred embodiment, the cheese substitute has the essential features of soft cheese or semi-soft cheese. Soft cheese contains between 67 and 80% moisture, on a fat-free basis, and can be bloomy rind or washed rind. Their protein content is often in the range of 20% of total weight. The soft cheese, bloomy rind has a rich and creamy texture with a slight elasticity in the cheese. The aging process depends on its thickness. This cheese has a mixed coagulation with slow draining, inoculated with specific molds. The soft, washed rind cheese has a rich and creamy texture with a slight elasticity in the cheese. During the aging process, the cheese is turned over regularly and brushed or washed in brine with beer, mead, wine or spirits. The semi-soft cheeses contain between 62% and 67% moisture, on a fat-free basis. The texture can be soft and creamy. When aging, the cheese can be washed (washed rind) in brine with red smear (with or without alcohol). The cheese can also be brushed and/or develop a natural rind.

In particular, in this embodiment, the edible composition
i. has a protein content comprised between 15 and 25% (weight content)
ii. has a moisture content comprised between 62% and 80%, on a fat-free basis

This can be achieved in particular when the LpCC obtained in b)
i. has a protein content comprised between 10 to 18% (weight content)
ii. has a moisture content superior to 62%, on a fat-free basis

In this embodiment, it is preferred when agar-agar is added to the LpCC in an amount to represent 0,5% to 1% (w/w) of the LpCC.

In this embodiment, it is advantageous when ferments for maturation [ripening ferments] are added in c), and wherein d) comprises salting and molding the curd.

In particular, step d) comprises maturing the curd at a temperature allowing for the development of the ferments for maturation, in particular at 14°C. The duration of maturation is determined by one of skill in the art depending on the aspect desired for the edible composition. It can be a few days or a few weeks.

Some other characteristics of the edible composition or of the LpCC used during the process for making the edible composition are described below, and can be found alone or in composition:
- the edible composition has a lipid content comprised between 10 and 30% (weight content)
- the LpCC has a lipid content comprised between 10% and 30 % (weight content)
- the edible composition has a calcium content lower than 2,8 % (weight content)
- LpCC has a calcium content comprised between 0,3 to 1,7% (weight content).

In one specific embodiment, is disclosed a method for the production of an edible composition comprising:
a) Providing a composition of casein, comprising recombinant casein as herein disclosed (preferably only recombinant casein as herein disclosed, preferably only casein of non-animal origin)
b) Mixing said composition of casein with at least one other ingredient comprising at least one component selected from the group consisting of water, calcium, lipids, and carbohydrate, so as to obtain a liquid pre-curd composition (LpCC),
c) Adding at least one curdling agent to the liquid pre-curd composition so as to obtain a curd
d) Further processing the curd to obtain an edible composition,
wherein a gelling agent is added in b), and wherein ferments for maturation are added in c), and wherein d) comprises salting and molding the curd.

In this embodiment, it is preferred when the edible composition
i. has a protein content comprised between 15 and 25% (weight content)
ii. has a moisture content comprised between 62% and 80%, on a fat-free basis.

This embodiment is well adapted for the production of a soft cheese substitute.

In addition, is disclosed a process comprising:
i) providing a bacterial composition, wherein the bacterial composition comprises bacteria that have been transformed with at least one nucleic acid coding for the casein, and the bacteria have been cultured so as to express and produce casein
ii) heating the bacteria at a temperature inducing enrichment of casein in the soluble fraction, and/or reduction of the amount of the other proteins in soluble fraction
iii) isolating the soluble fraction after heating
iv) optionally, further processing said soluble fraction by adding at least one step chosen among addition of activated charcoal, membrane filtration, chromatography or casein precipitation, thereby obtaining a casein composition
v) Mixing said casein composition with at least one other ingredient comprising at least one component selected from the group consisting of water, calcium, lipids, and carbohydrate, so as to obtain a liquid pre-curd composition (LpCC)
vi) Adding at least one curdling agent to the liquid composition so as to obtain a curd.

When the bacteria are not lysed, heating in ii) shall be performed at a temperature inducing lysis of the cells, thereby producing a bacterial extract.

In a preferred embodiment, said curdling agent is not rennet, but is an acidifying agent. In a preferred embodiment, said curdling agent is a lactic bacteria or lactic, citric or acetic acid.

The term "liquid pre-curd composition" or "LpCC" designates the composition containing at least a casein and at least one other ingredient comprising at least a component among water, calcium, lipids, an carbohydrate, before the addition of rennet and ferments and curdling (in particular coagulation of casein).

"Curdling agent" designates a chemical or biochemical composition capable of triggering curdling. Curdling can be achieved by the addition of acid solutions, by the addition of starter cultures (which growth in the presence of carbohydrates triggers a decrease of pH, by the addition of natural or recombinant rennet, by the addition of rennet substitutes, such as animal proteases, or vegetal curdling enzymes), or by a combination of these processes. It is also possible to perform heat treatment, or to add a calcium chelating agent, in particular to improve or speed up curdling.

A curdling agent may be any additive, compound, composition or treatment which addition results in curdling, alone or in combination with another or other additives, compounds, compositions or treatments.

The global protein content (including caseins and derived peptides) is dependent on cheese type and production process. Protein content in the LpCC will impact the protein content in the final product, and can be adjusted in order to produce a substitute for a desired type of cheese. Protein content in the LpCC (weight content), corresponds to the mass of protein in the LpCC divided by the total mass of the LpCC

In a preferred embodiment, protein content in the LpCC is inferior to 12% (weight content). In a more preferred embodiment, it is comprised between 2% and 10% (weight content). In a more preferred embodiment, it is comprised between 5% and 10% (weight content). These amounts are particularly interesting for the production of a substitute for fresh cheese.

In another preferred embodiment, protein content in the LpCC is comprised between 10% and 25% (weight content). In a more preferred embodiment, it is comprised between 10% and 18% (weight content). These amounts are particularly interesting for production of a substitute for a soft or semi-soft cheese.

### Calcium and other salts

Cow's milk contains about 1,2g of Calcium per liter (about 0,12%, weight content). Calcium is present in cheese, contributing to its nutritive qualities. Calcium represents in the range of 0.1 to 1% of cheese's weight, with the lowest contents of calcium being usually found in fresh cheeses (0.125%, weight content in french "fromage blanc"), while the highest contents are usually found in firm and hard cheese (0.97% in Emmental). In camembert, it is in the range of 0.25%

(weight content). In the cheese production process, the amount of calcium remaining in milk depends on the process (Mietton et Chablain, Du lait au fromage: les fondamentaux technologiques. In Gillis JC, Ayerbe A, Le fromage 4ème edition, Lavoisier - Technique Et Documentation 20 avril 2018, with a better retention being achieved with rennet (62-67% of calcium) than with lactic curdling agents (16-17% of calcium). In addition, in order to recapitulate closely the properties of a given cheese category, the percentage of calcium in the LpCC will be important. However, cheese with enhanced calcium content can be envisioned.

In a preferred embodiment, calcium content in the LpCC is comprised between 0.1% and 1.7%.

Some food additive with calcium, notably calcium carbonate (E170) are of animal origin. However, calcium of mineral or vegetal (from the lithothamne sea weed for example) origin also exist. The methods disclosed herein offer the possibility to substitute milk's calcium with calcium of non-animal origin, thereby allowing for diminution of the components of animal origin. In a more preferred embodiment, calcium is from non animal-origin. Other salts can be added, such as, phosphate which is an important component of milk, or others.

### Lipids

Lipids are present in most cheeses, and play a role in the organoleptic sensation. However, cheese with reduced fat content (low fat) or even with no fat have been produced during the last decades, in order to address health concerns. The method disclosed herein offers the possibility to substitute milk lipids with lipids of non-animal origin, thereby allowing for diminution of the components of animal origin. In a more preferred embodiment, said lipids are lipids extracted from plant.

In cheese, fat content may vary from 0% in a totally fat-free product, to more than 30%. Fat content in a cheese corresponds to the mass of lipids in the cheese divided by the total mass of the cheese.

Fat composition may be adjusted, in quantity and quality, in order to mimic the composition of an existing cheese. Lipids are present in high amount in most cheese: 95%-10% of milk's lipids remain in the curd during curdling. However, lipid content varies a lot depending on milk's origin (cow, goat, sheep), and on milk conditioning and cheese production process.

It can be only 7% in fresh cheese, or even lower, and higher than 30% in traditional hard cheese. Recent industrial cheeses include products with very low lipid contents, in order to address a demand for fat-free products, or very high lipid levels (36% of total weight in Boursault, made by Bongrain, France, for example).

In the context of the present description, fat composition in the LpCC may be modified at will, in order to obtain fat-free or fat-rich products. Fat content in the LpCC (weight content), corresponds to the mass of lipids in the LpCC divided by the total mass of the LpCC.

In particular, fat composition in the LpCC is 0% (weight content). In another preferred embodiment, it is lower than 10% (weight content). In another preferred embodiment, it is comprised between 10% and 20% (weight content). In another preferred embodiment, it is comprised between 20% and 30% (weight content). In another preferred embodiment, it is comprised between 30% and 40% (weight content).

In milk, lipids consist essentially in triglycerides, i.e., molecules resulting from the fusion of a glycerol molecule ad three fatty acids, by esterification, but this composition will vary a lot during cheese processing, with the hydrolysis of triglycerides producing various amounts of mono and diglycerides, as well as free fatty acids. Free fatty acids can be further processed and have a very important role in the taste and smell of the final product.

Lipids may be included as a composition of lipids extracted from plant. The major fatty acids in cow milk fat are palmitic acid (31%), oleic acid (24%), myristic acid (12%), stearic acid (11%), lower size saturated acids (11%), palmitolic acid (4%) linoleic acid (3%), trans-unsaturated acids (3%) and alpha-linoleic acid (1%). Such composition can be recapitulated in the LpCC. However, it can also be modulated, for example to increase the proportion of unsaturated fatty acids.

Dairy products are rich in saturated fatty acids: in milk, the saturated palmitic, myristic and stearic acids, together with lower size molecules, represent 65% of the fatty acids. One of the advantages of using for example a lipid composition from plants could provide a healthier source of fat. Indeed, plant fat usually contains less saturated fatty acids and trans-fatty acids than animal fat. In addition, the composition could be reinforced in lipids known to have health benefits, such as omega-3 fatty acids, including for example alpha-linolenic acid, eicosapentaenoic acid or docosahexaenoic acid.

In the context of the present description, various lipid sources form plant can be used, such as canola or rapeseed, sunflower, soybean, coco oil, olive oil, walnut oil, hazel nut oil, and margarin. Such products are largely commercialized. However, in products of sufficient purity should be used, in order to avoid the carryover of undesired flavors and/or tastes. Deodorized oils can be used. Deodorization is a steam stripping process wherein a good-quality steam, generated from de-aerated and properly treated feedwater, is injected into soybean oil under low absolute pressure and sufficiently high temperature to vaporize the Free Fatty Acid (FFA) and odoriferous compounds and carry these volatiles away from the feedstock. The various processes of soybean oil processing are described in Practical Handbook of Soybean Processing and Utilization (1995), OCS Press. Elsevier Inc.

In particular, lipids consist in in canola oil, rapeseed oil, sunflower oil, soybean oil, coco oil, olive oil, walnut oil, hazel nut oil, or margarin. In a more preferred embodiment, lipids consist in canola oil, rapeseed oil, soybean oil or coco oil.

### Emulsifying and gelling agents

An emulsifier or emulsifying agent is a compound or substance that acts as a stabilizer for emulsions, preventing liquids that ordinarily don't mix from separating. It can be helpful using an emulsifying agent, in order to avoid formation of a solid and liquid phase during and after the preparation of the LpCC. A very widespread is lecithin. The term actually designates a family of amphiphilic compounds, found in animal and plants, but commercial soybean or sunflower lecithin can easily be found.

In particular, an emulsifying agent is added, in order to avoid the formation of a liquid and a solid phase. In a more preferred embodiment, this emulsifying agent is lecithin. An emulsifying agent can be added in step b) or in the LpCC before (or while) adding the curdling agent in c) (however, the emulsifier should be mixed with the LpCC before curdling).

Alternatively, or in addition, gelling agents such as agar-agar or guar gum, arabic gum, tragacanth gum, xanthan gum, carob (locust bean) gum, cellulose gum, cassia gum, sesbania gum, tamarind gum, fenugreek gum, carrageenans, could be used. Such hydrocolloids add firmness and enhancing water retention. Gelling agents of animal origin, such as gelatin, should be excluded from the composition.

Addition of gelling agent before curdling or after curdling (mixed with the curd) is of particular interest in the context of the description. This would help provide texture to the edible composition. one can use, in particular locust bean gum.

Agar-agar (which would be added to the LpCC in an amount to represent 0,5% to 0,8% (w/w) of LpCC, or 0,5% to 1% (w/w) of the LpCC) is particularly interesting.

### Carbohydrates

In particular, the other ingredients contain at least calcium, lipids, and a carbohydrate. Lactose is the major milk carbohydrate. Cow's milk contains 70 mg/L of lactose, 20 mg/L of galactose and traces of other carbohydrates including various oligosaccharides. During and in aged cheese it is eventually totally or almost totally degraded. However, it is still present in significant amounts in fresh cheese, which should usually be avoided by people with strong lactose intolerance. Anyway, for each cheese type, individual reaction will depend on the severity of the lactose intolerance.

Lactose is important because it is a feedstock for cheese microflora (ferments). However, it can be replaced by glucose or other carbohydrates. Carbohydrates such as glucose, saccharose or fructose are derived from plants, whereas most commercial lactose is today of animal origin. The methods disclosed herein offer the possibility to substitute milk's lactose with glucose or with another carbohydrate of non-animal origin, thereby allowing for diminution of the components of animal origin, with the additional advantage of definitively dealing with lactose intolerance.

In particular, no lactose is added in the composition. In a more preferred embodiment, a carbohydrate of non-animal origin is added in the composition. In an even more preferred embodiment, this carbohydrate is glucose, saccharose or fructose.

### Vitamins

In particular, the other ingredients contain at least calcium, lipids, a carbohydrate, and a vitamin. Dairy products are usually rich in vitamins. Milk is a good source of hydrosoluble vitamins B₁₂, B₂, B₈, B₅, but contribute little to the needs for vitamin B₃. Milk also brings the liposoluble vitamin A but is a poor source of vitamins D, E and K. However, in cheese, which is usually enriched in fat, this low content in liposoluble vitamins is alleviated. Cheese substitute may have the same nutritional value in terms of vitamin as conventional cheese, and may be even supplemented with higher vitamin amounts. However, in the context of a need for substitutes of non-animal origin, which may often be associated with a vegan diet, or with a diet with a lower animal product content, vitamin B₁₂ is the most important additive to provide, since it is usually brought essentially by food derived from animals. In a more preferred embodiment, the composition is supplemented with at least a vitamin. In an even more preferred embodiment, this vitamin is vitamin B₁₂.

In particular, the other ingredients contain at least calcium, lipids, a carbohydrate, and vitamin. B12.

### Curdling agents

Whereas milk clotting can be achieved by the addition of acid, or simply by lactic fermentation, most cheese today are made using rennet, most of the time ion conjunction with lactic bacteria. Upon digestion of κ-casein by rennet enzymes, casein/calcium phosphate micelles are destabilized, and clotting occurs, resulting in the formation of curd. Thus, rennet will be used in most cases. However, different types of rennet exist, as described above. Traditional rennet are being derived from calves stomach, but other types of rennet have been developed, including plant-based rennet and rennet derived from recombinant microorganisms. Such non animal rennet can be bought for example from Chr. Hansen Lab. (DK), Gist Brocades/DSM. (NL), Pfizer Inc. (USA) (see Le fromage Gillis JC, Ayerbe A (2018), Lavoisier ed., Paris).

In particular, a curdling agent of non-animal origin is added. In a preferred embodiment, said curdling agent is not rennet, but is an acidifying agent. In a preferred embodiment, said curdling agent is a lactic bacteria or lactic, citric or acetic acid.

### Ferments

In particular, a ferment is added. Lactic bacteria are essential components of cheese ferments. They contribute to lactic acid production, which in turn modify the pH, thereby modifying the physico-chemical conditions, and contributing, together with rennet, to the texture of the final product. In an even more preferred embodiment, this ferment comprises at least one lactic bacteria. In an even more preferred embodiment, lactic bacteria is chosen among *Streptococcus cremoris, Streptococcus lactis, Streptococcus diacetylactis, Streptococcus thermophilus, Leuconostoc lactis, Leuconostoc citrovorum, Lactobacillus bulgaricus,* and *Lactobacillus helveticus.*

Lactic bacteria are the first microbes to spread in milk and curd, and by modifying the composition of curd, pave the way for the next wave of ferments. Together with this next wave of ferments, they contribute to maturation or aging, including non-starter lactic acid bacteria (NSLAB) and other microorganisms. In an even more preferred embodiment, the ferments comprise at least one NSLAB. In an even more preferred embodiment, this NSLAB is selected from the group consisting of *Lactobacillus paracasei, Lactobacillus casei, Lactobacillus ramnosus, Lactobacillus plantarum, Lactobacillus brevis, Lactobacillus bucheneri, Lactobacillus fermentum, Enterocuccus faecalis, Enterocuccus faecium, Pediococcus pentosaceus,* and *Carbiobacterium laltaromatium.*

In particular, the ferments contain at least another microbe which is not a lactic bacterium, and which can be a yeast, a mold, or a non-lactic bacterium. In an even more preferred embodiment, the ferments contain at least another microbe, which is a yeast selected from the group consisting of *Debaryomyces hansenii, Geotrichum candidum, Kluyveromyces larxianus, Kluyveromyces lactis, Saccharomyces cerevisae, Candida catenulate, Candida intermediata, Torulaspora delbrueckii, Candida zeylanoides, Pichia mebranifaciens, Candida rugiosa,* and *Pichia fermentans.*

In particular, the ferments contain at least another microbe, which is mold selected from the group consisting of *Penicillium camemberti, Penicillium roqueforti, Chrysosporum sulfureum, Fusarium domesticum, Isomucor fuscus, Mucor plumbeus, Penicillium commune,* and *Sprendonema casei.*

Ferments can be commercially obtained from a large number of companies, including for example Chr. Hansen.

### Water (moisture)

In cow's milk, water represents about 87.4% (weight content), and 90.8% on a fat free basis. Water content, weight content, is the mass of water divided by the total weight, in a given volume. Water content, also called moisture, on a fat-free basis, is the mass of water present in the product, divided by the total mass minus the mass of fat (=mass water/(total mass-mass of fat). Moisture is an important feature of cheese type, and is major criteria of the cheese types as defined below.

Ideally, moisture in LpCC should as low as possible, in order to avoid waste, while still allowing some draining. In the case of soft or semi-soft cheese and firm or hard cheese (see below), LpCC could have moisture contents significantly below the moisture content of milk.

In particular, moisture content in the LpCC is inferior to 90%, on a fat free basis. In a more preferred embodiment, it is below 80%.

When using desiccated casein, one can add water and fat, as well as an emulsifier agent in order to obtain the LpCC. As an illustration, if one is to use desiccated casein containing about 23 % casein, water and other elements (such as carbohydrates), one can add, for 1 g of desiccated casein, 1.3 g of water, about 2 g of fat, and about 0.027 g of emulsifier. pH can be adjusted to about 5-5.5 with any appropriate acidic agent (lemon juice can be used, but also lactic, citric or acetic acid).

Fat is preferably a vegetable fat, such as peanut oil, coconut oil, canola oil, hazelnut oil, walnut oil, avocado oil, olive oil. It is preferred to use a neutral oil *(i.e.* an oil that doesn't have a taste or odor). In particular, deodorized coconut oil can be used.

The emulsifier is selected from the list of such emulsifiers authorized for food use. One can cite lecithins (in particular soy lecithin), propylene glycol esters of fatty acids (PGMS), mono and diglycerides.

### Processing of the curd

The curd can then be further processed, to obtain a cheese substitute.

Such processing of the curd include one or more steps such as
- cutting the curd and heating it
- draining the curd to remove water (this corresponds to the removal of whey in the traditional cheese processing using milk), so as to allow the curd to form a mat
- texture the curd: it may be cut into section, piled, flipped, pressed so as to expel more water in case it is needed or allow fermentation to continue
- dry and/or salt and/or brine to product
- form blocks of cheese substitute.

The substitute can be stored and allowed to age, in particular to allow taste and texture to mature and develop.

The processing of the curd is essentially similar to the processing of a curd for making cheese.

### DESCRIPTION OF THE FIGURES

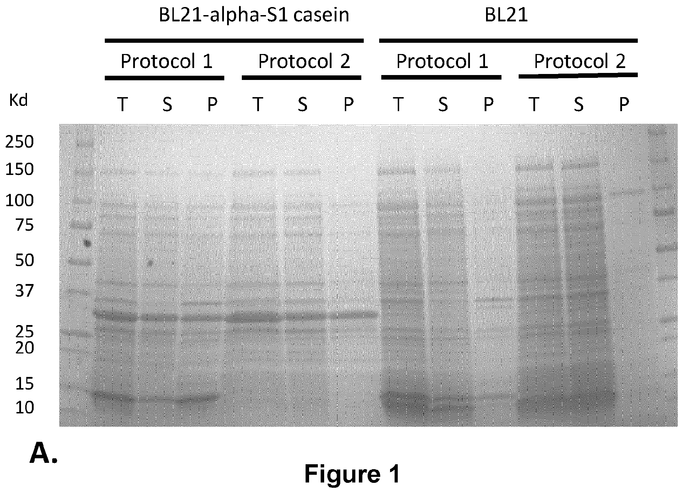

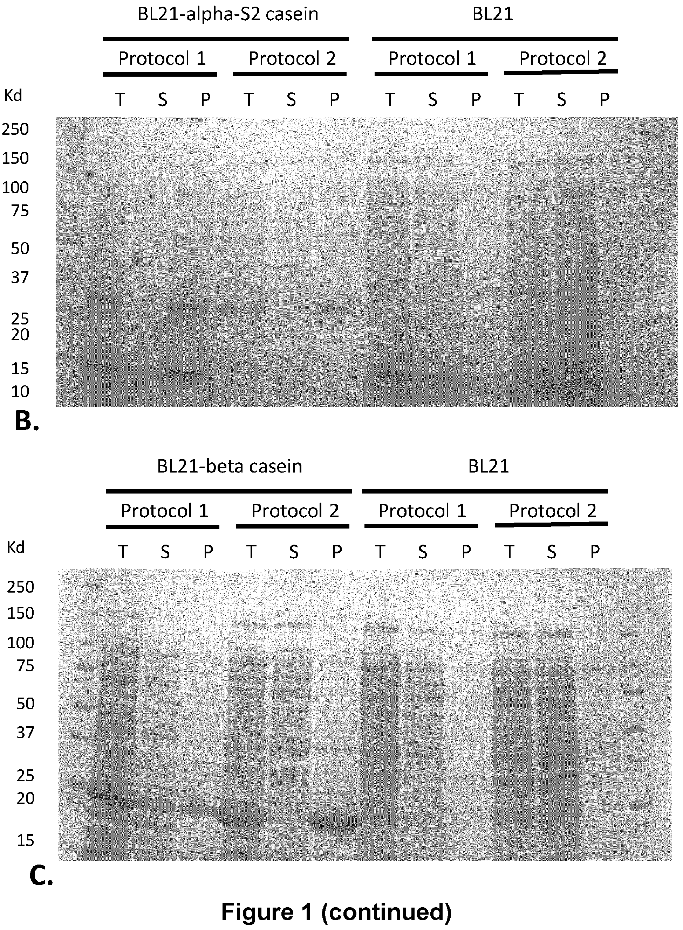

**Figure 1****:** Analysis by SDS-PAGE of soluble and insoluble fractions of extracts from recombinant cells expressing alpha-S1 casein **(A),** alpha-S2 casein **(B)** and beta casein (C). Cells transformed with vector expressing casein (BL21-alpha-S1 casein, BL21-alpha-S2 casein or BL21-beta casein) or empty vector (BL21) were lysed with two different protocols described in Example 1. Lysates were centrifuged and analyzed by SDS-PAGE. T: total (before centrifugation), S: supernatant, P: pellet.

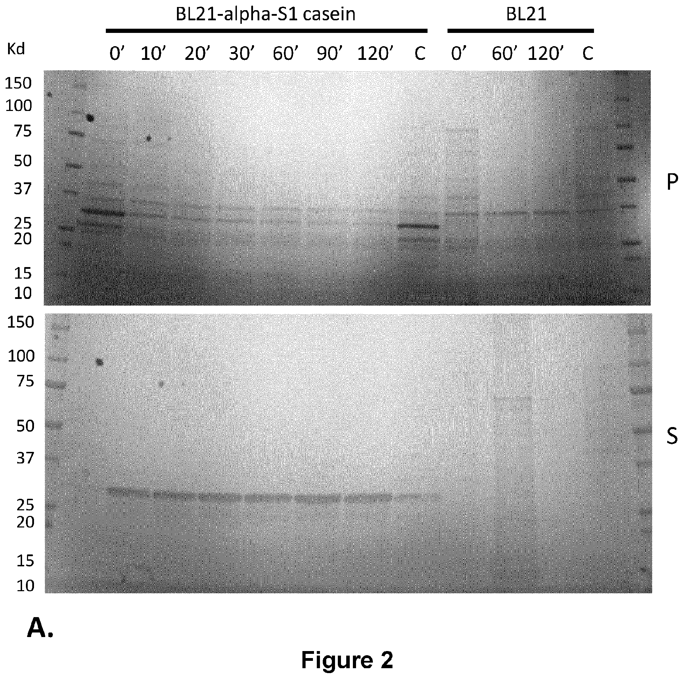

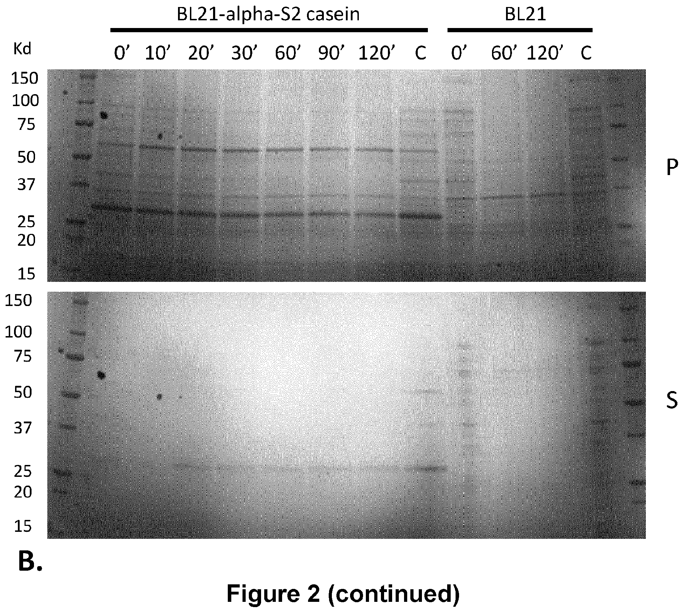

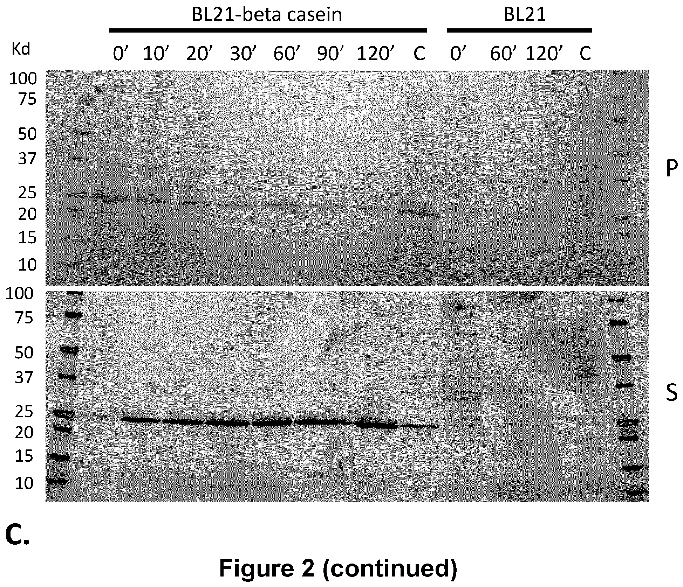

**Figure 2****:** SDS-PAGE analysis of soluble and insoluble fractions of heat-treated extracts from recombinant cells expressing alpha-S1 casein **(A),** alpha-S2 casein **(B)** and beta casein (C). Cells transformed with vector expressing casein (BL21-alpha-S1 casein, BL21-alpha-S2 casein or BL21-beta casein) or empty vector (BL21) were heated for 10, 20, 30, 60, 90, or 120 minutes (10'; 20'; 30';60';90';120'; 0': non heated samples; C: samples incubated at room temperature for 120 minutes). Lysates were centrifuged and analyzed by SDS-PAGE. T: total (before centrifugation), S: supernatant, P: pellet. See Example 2 for details.

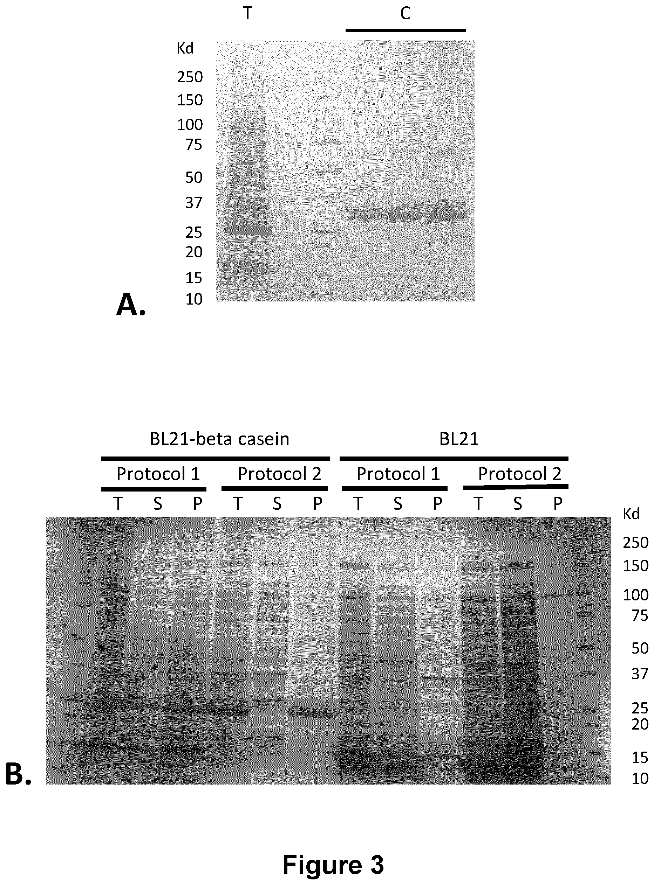

**Figure 3****:** SDS-PAGE analysis of beta casein produced in E. coli in a 42L fermenter. **A.** Analysis of total extract. T: total extract (5' heating at 95°C in Laemmli loading buffer). C: beta casein Sigma (5, 10 and 20 µg of protein were loaded in the three different lanes). See Example 3 for details. **B.** Analysis of soluble and insoluble fraction. For details, see Example 3.

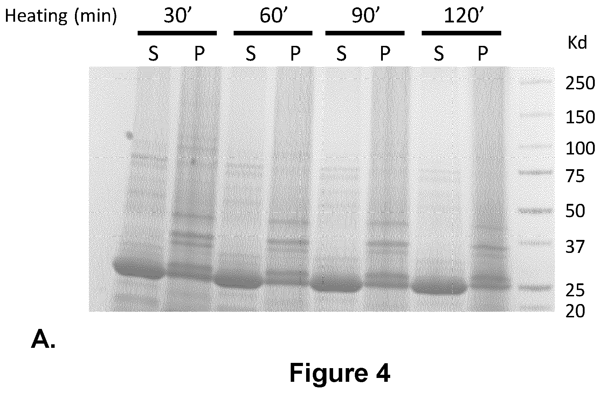

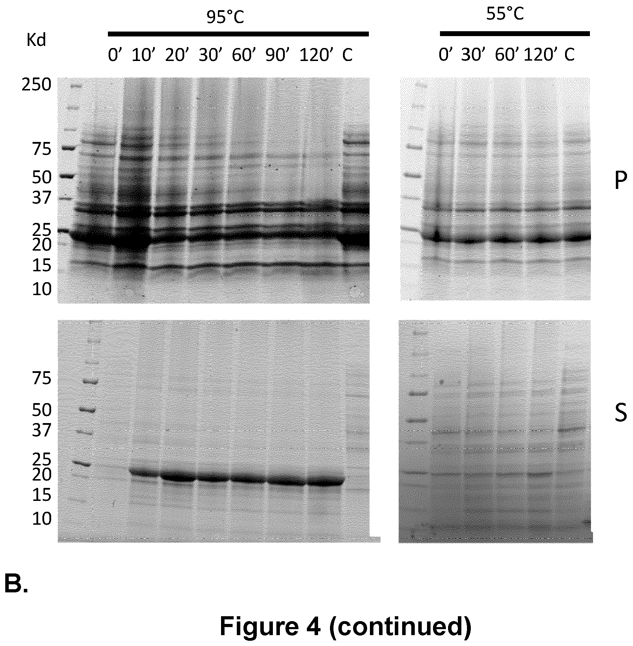

**Figure 4****:** SDS-PAGE analysis of soluble and insoluble fractions of heat-treated extracts from recombinant cells expressing beta casein, grown in a 42L bioreactor. See Example 4 for details. **A:** Cells transformed with vector expressing beta casein were heated at 95°C for 30, 60, 90, or 120 minutes (30';60';90';120'). Lysate were centrifuged and the soluble and insoluble fractions were analyzed by SDS-PAGE. S: supernatant; P: pellet. **B.:** Cells transformed with vector expressing beta casein were heated at 55°C for 30, 60, 90, or 120 minutes (30';60';90';120'), or at 95°C for 10, 20, 30, 60, 90, or 120 minutes (10; 20; 30';60';90';120'). Lysates were centrifuged and both the soluble and insoluble fractions were analyzed by SDS-PAGE. 0': non heated samples; C: samples incubated at room temperature for 120 minutes.

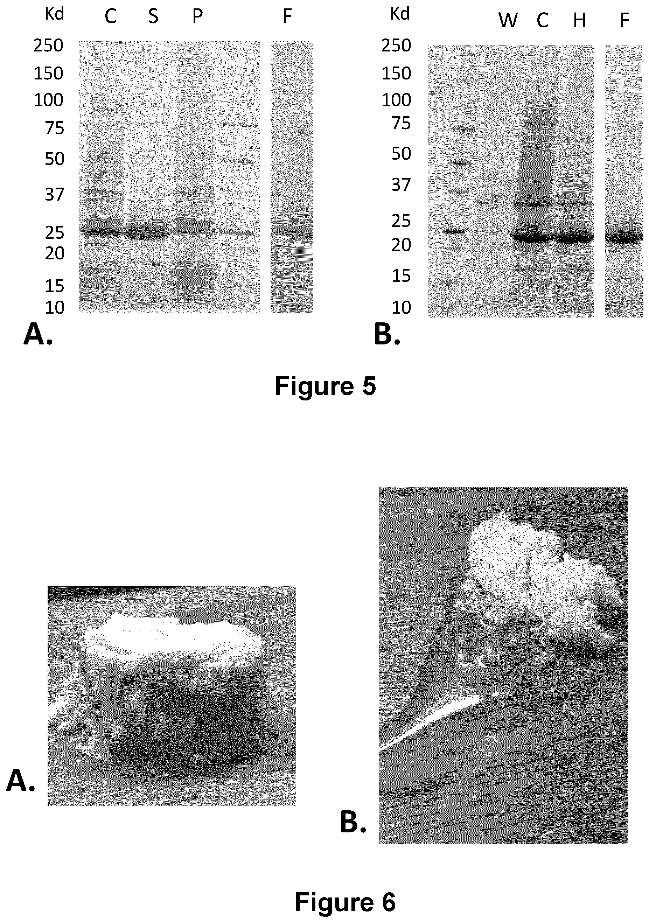

**Figure 5****:** Monitoring by SDS-PAGE of beta casein produced in *E. coli,* and partially purified. **(A)** Samples were prepared as described in Example 5, by recovering and resuspending cells, heating them 90 minutes at 95°C, and further processing by centrifugation and filtration. **(B)** Samples were prepared as described in Example 5, in order to compare resuspended cell pellets before and after heating (lysate). A filtered sample was also monitored. W: washing solution; C: resuspended cell pellet; H: lysate after heating S: lysate supernatant after centrifugation; P: lysate pellet; F: final filtered sample.
**Figure 6****:** Making of a curd from partially purified beta casein. The partially purified preparation of beta casein described in Example 5 was desiccated by heat and mixed with other ingredients as described in Example 6. The resulting mixture formed a curd upon addition of lemon, molding and incubation **(A).** A mixture made with the same ingredients but casein. This control mixture partly solidified upon cooling, but forming crumble, and bad water retention as observed **(B).**

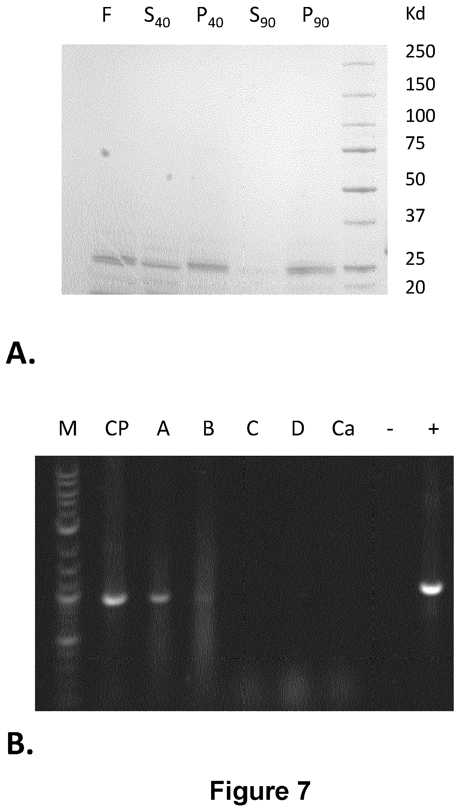

**Figure 7****:** Purification of beta casein by thermo lysis and precipitation in acidic conditions. Samples were treated and analyzed as described in example 7. **A.** Monitoring of beta casein by SDS-PAGE. Beta casein was monitored after thermal lysis for two hours at 95°C, supernatant isolation and filtration (S), and after pH adjustment of this supernatant at pH=4, heating 20 minutes at 40°C or 90°C, and centrifugation for 20' at 3220 g. After this last centrifugation step, both supernatant and pellet were analyzed: supernatant after heating at 40°C (S40), pellet after heating at 40°C (P40), supernatants after heating at 90°C (S90), pellet after heating at 90°C (P90). **B.** Monitoring of recombinant DNA in resuspended cell pellet (CP) and at various steps of the purification treatment, including: in the supernatant after heating at 95°C for 2 hours (lane A), after pH adjustment of this supernatant at pH4 (lane B), after further heating at 90°C for 20 minutes, centrifugation and resuspension of the precipitated caseins (C), after washing of the precipitated caseins and resuspension (D), after Ca(OH)₂ addition (Ca). Control PCR were made in the absence of any DNA or cell samples (-) or in the presence of recombinant plasmid expressing beta casein.(+). M: 1kb plus NEB marker.

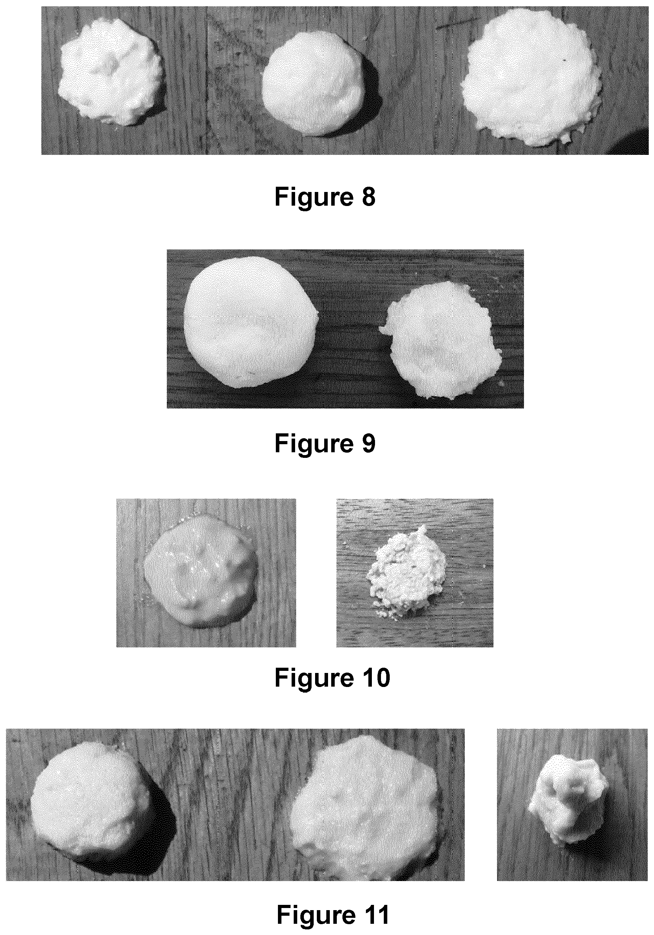

**Figure 8****: curdling with calcium caseinates and a gelling agent.** LpCC was seeded in the presence or absence of a gelling agent (agar-agar). Compositions and protocol are described in example. Preparations are represented at the end of the draining step. Left: curdling in the absence of gelling agent. Middle: curdling in the presence of 0.68% agar-agar. Right: 0.68% agar-agar, no curdling agent.
**Figure 9****: curdling with calcium caseinates and addition of a gelling agents added after curdling.** Compositions and protocol are described in example 6. Preparations are represented after the end of the draining step. Left: 0.53% agar-agar is added after curdling. Right: 0.53% agar-agar is added in the absence of curdling.
**Figure 10****: Coagulation of recombinant caseins.** Compositions and protocol are described in example 10. Left: curdling with beta casein. Curdling was made in the presence of lipids and carbohydrates, but no gelling agent. Preparations is represented at the end of the draining step. Right: coagulation of alpha-S1 and beta caseins at acidic pH. No lipids or carbohydrates were added to the casein composition. Left and right pictures are not at scale.
**Figure 11****: Making a soft cheese from recombinant caseins.** Compositions and protocol are described in example 11. Preparations are represented at the end of the draining step. Left: curdling of beta casein (Batch 1) in the presence of 0.68% agar-agar. Middle: curdling of beta casein,0.68% agar-agar, no curdling agent. Right: curdling of alpha-S1 and beta casein in the presence of 0.68% agar-agar.

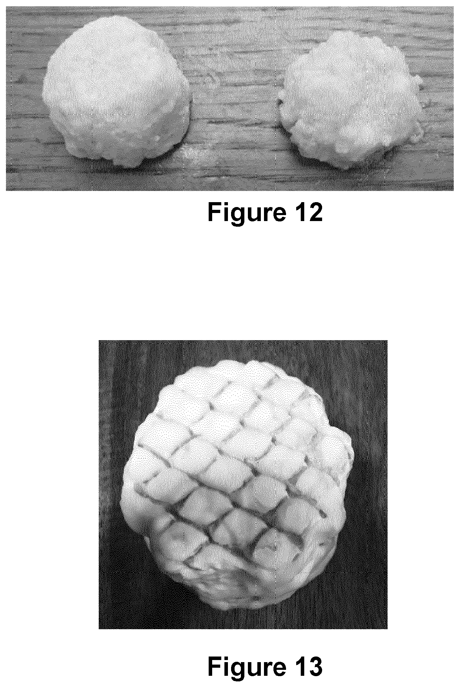

**Figure 12****: Addition of a gelling agents added after curdling of beta casein.** Compositions and protocol are described in example 11. Preparations are represented after the end of the draining step. Left: 0.53% agar-agar is added after curdling. Right: 0.53% agar-agar is added in the absence of curdling.
**Figure 13****: Soft cheese substitute made from recombinant beta casein.** The product was made as described in example 12. The product is represented after 7 days of aging.

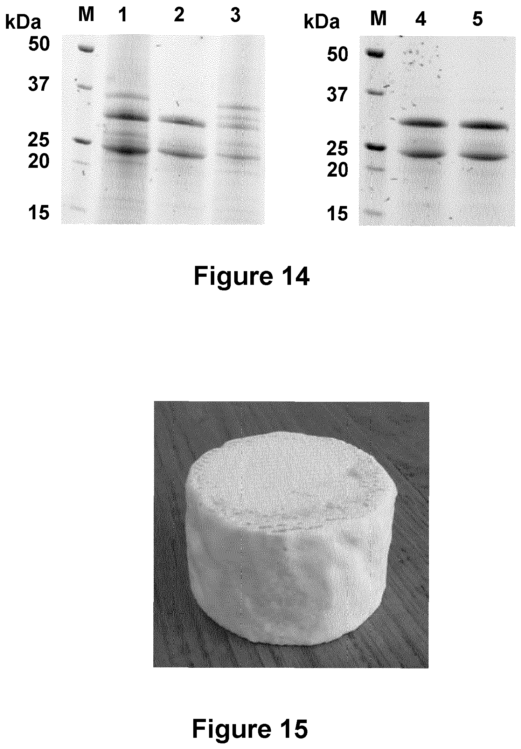

**Figure 14****: SDS-PAGE analysis of the alpha-S1 and β caseins at various steps of the purification process.** Samples were treated and analyzed as described in example 13. **A: M: Molecular weight marker Lane 1:** Lysate; Lanes 2 and 4: Clarified lysate, Lane 3 : Insoluble pellets; Lane 4: clarified Lysate after treatment with activated charcoal. M: molecular weight marker. Band sizes were indicated left of the gel.
**Figure 15****: Cheese substitute.** Cheese substitute was produced as described in Example 14. Picture was taken 6 days after curdling.

### EXAMPLES

### Example 1: Monitoring of recombinant caseins in the soluble and insoluble fraction of E. coli extracts (not according to the invention as defined by the claims)

Natural casein genes code for a precursor protein, which includes a signal peptide.

In mammals, this peptide is cleaved during casein processing, and is not present in the mature protein in milk. Synthetic genes coding for alpha-S1, alpha-S2 and beta casein (related to natural genes P02662, P02663 and P02666, respectively), were modified, in order to remove the signal peptide, and the sequence of the new synthetic open reading frames are shown in Table 1, last column). They were cloned into pET25b+ and the resulting plasmids were transformed into BL21(DE3) strains. Individual transformed clones were isolated, and for each synthetic gene, one clone was used to inoculate LB medium. A clone transformed with empty vector (pET25b+) was used as control.

**Table 1: Sequences of natural caseins (precursors) and of the related recombinant proteins. In natural caseins, signal peptides are indicated in bold.**

| Name and Uniprot reference of protein precursor | sequence of protein precursor | sequence of recombinant proteins in examples |
|---|---|---|
| Alpha-S1 casein P02662 | MKLLILTCLVAVALARPKHPIK HQGLPQEVLNENLLRFFVAPFP EVFGKEKVNELSKDIGSESTED QAMEDIKQMEAESISSSEEIVP NSVEQKHIQKEDVPSERYLGYL EQLLRLKKYKVPQLEIVPNSAE | MRPKHPIKHQGLPQEVLNENLLR FFVAPFPEVFGKEKVNELSKDIG SESTEDQAMEDIKQMEAESISSS EEIVPNSVEQKHIQKEDVPSERY LGYLEQLLRLKKYKVPQLEIVPNS AEERLHSMKEGIHAQQKEPMIGV |
| | ERLHSMKEGIHAQQKEPMIGV NQELAYFYPELFRQFYQLDAY PSGAWYYVPLGTQYTDAPSFS DIPNPIGSENSEKTTMPLW (SEQ ID NO: 1) | NQELAYFYPELFRQFYQLDAYPS GAWYYVPLGTQYTDAPSFSDIPN PIGSENSEKTTMPLW (SEQ ID NO: 2) |
| Alpha-S1 casein A0A3Q1M JE5 | MKLLILTCLVAVALARPKHPIK HQGLPQEVLNENLLRFFVAPFP EVFGKEKVNELSKDIGSESTED QAMEDIKQMEAESISSSEEIVP NSVEQKHIQKEDVPSERYLGYL EIVPNSAEERLHSMKEGIHAQQ KEPMIGVNQELAYFYPELFRQF YQLDAYPSGAWYYVPLGTQYT DAPSFSDIPNPIGSENSEKTTM PLW (SEQ ID NO: 3) | |
| Alpha-S1 casein A0A3Q1N G86 | MKLLILTCLVAVALARPKHPIK HQGLPQEVLNENLLRFFVAPFP EVFGKEKVNELSKDIGSESTED QAMEDIKQMEAESISSSEEIVP NSVEQKHIQKEDVPSERYLGYL EQLLRLKKYKVPQLERLHSMK EGIHAQQKEPMIGVNQELAYFY PELFRQFYQLDAYPSGAWYYV PLGTQYTDAPSFSDIPNPIGSE NSEKTTMPLW (SEQ ID NO: 4) | |
| Alpha-S2 casein P02663 | MKFFIFTCLLAVALAKNTMEHV SSSEESIISQETYKQEKNMAINP SKENLCSTFCKEVVRNANEEE YSIGSSSEESAEVATEEVKITVD DKHYQKALNEINQFYQKFPQYL QYLYQGPIVLNPWDQVKRNAV PITPTLNREQLSTSEENSKKTV DMESTEVFTKKTKLTEEEKNRL NFLKKISQRYQKFALPQYLKTV YQHQKAMKPWIQPKTKVIPYV RYL (SEQ ID NO: 5) | MKNTMEHVSSSEESIISQETYKQ EKNMAINPSKENLCSTFCKEVVR NANEEEYSIGSSSEESAEVATEE VKITVDDKHYQKALNEINQFYQK FPQYLQYLYQGPIVLNPWDQVK RNAVPITPTLNREQLSTSEENSK KTVDMESTEVFTKKTKLTEEEKN RLNFLKKISQRYQKFALPQYLKT VYQHQKAMKPWIQPKTKVIPYVR YL (SEQ ID NO: 6) |
| Beta casein P02666 | MKVLILACLVALALARELEELN VPGEIVESLSSSEESITRINKKIE KFQSEEQQQTEDELQDKIHPF AQTQSLVYPFPGPIPNSLPQNI PPLTQTPVVVPPFLQPEVMGV SKVKEAMAPKHKEMPFPKYPV EPFTESQSLTLTDVENLHLPLP LLQSWMHQPHQPLPPTVMFPP QSVLSLSQSKVLPVPQKAVPY PQRDMPIQAFLLYQEPVLGPV RGPFPIIV (SEQ ID NO: 7) | MRELEELNVPGEIVESLSSSEESI TRINKKIEKFQSEEQQQTEDELQ DKIHPFAQTQSLVYPFPGPIPNSL PQNIPPLTQTPVVVPPFLQPEVM GVSKVKEAMAPKHKEMPFPKYP VEPFTESQSLTLTDVENLHLPLPL LQSWMHQPHQPLPPTVMFPPQ SVLSLSQSKVLPVPQKAVPYPQR DMPIQAFLLYQEPVLGPVRGPFP IIV (SEQ ID NO: 8) |
| Kappa casein P02668 | MMKSFFLVVTILALTLPFLGAQ EQNQEQPIRCEKDERFFSDKIA KYIPIQYVLSRYPSYGLNYYQQ KPVALINNQFLPYPYYAKPAAV RSPAQILQWQVLSNTVPAKSC QAQPTTMARHPHPHLSFMAIP PKKNQDKTEIPTINTIASGEPTS TPTTEAVESTVATLEDSPEVIES PPEINTVQVTSTAV (SEQ ID NO: 9) | |

Cells were lysed using two different protocols, and total extracts as well as samples from soluble and insoluble fractions were analyzed by SDS page. Caseins were monitored in the soluble and insoluble fractions of cell extracts using two different protocols. Cells transformed with empty vector (pET25b+) were used as control.

### Protocol 1:

Cell pellets obtained from a 100 mL culture were lysed by resuspension in lysis buffer (50 mM Tris HCl pH 7.5, 1 mg/mL lysozyme, 0,03 mg/mL Dnase). The suspension was incubated on ice for 30 min and then sonicated for 10 seconds (10% amplitude, Q Sonica XL-2000). 10 µL of the total fraction, were taken and kept for SDS-PAGE analysis. The soluble and insoluble fraction were separated by centrifugation at 3220 g and 4°C for 20 min. The supernatant was recovered and supplemented with 10% glycerol for preservation. The pellet was further resuspended in Tris 50 mM with SDS 2% and supplemented with 10% glycerol for preservation. 10 µL of each fraction were taken for SDS-PAGE.

### Protocol 2:

Cell pellets obtained from a 100 mL culture were lysed by resuspension in Bugbuster (Millipore) with 0,4% Lysonase (Millipore). The mix was incubated at room temperature for 5 min and then centrifuged at 3220 g and 4°C for 20 min. The supernatant was recovered and 10 µL of the supernatant were taken and reserved for SDS-PAGE analysis. The pellet was further resuspended in SDS 2% and 10 µL of the suspension, representing the insoluble fraction, were then taken and preserved for SDS-PAGE analysis.

As shown on Figure 1, alpha-S1 casein was found in both the soluble (S) and insoluble (P) fractions (Fig. 1A), while alpha-S2 was found essentially in the insoluble fraction (Fig. 1B), and beta caseins largely (protocol 1) or totally (protocol 2) in the insoluble fraction (Fig. 1C), as often observed with overexpression of recombinant proteins in E. coli. This suggests that in E. coli, caseins may be present in inclusion bodies. The different quantitative outcomes can be due to different solubilities of beta casein or different stabilities of inclusion bodies depending on the protocol. Nevertheless, recovery of caseins from the soluble fraction of the extracts would result in loss of a large part or of all of the recombinant proteins using these protocols.

### Example 2: Monitoring of recombinant caseins in the soluble and insoluble fraction of E. coli extracts resulting from heating

The impact of lysis by heating on caseins was tested with the recombinant strains described in Example 1. A clone transformed with empty vector (pET25b+) was used as control.

Cultures were centrifuged and cell pellets were resuspended in 1 volume of sterile water, centrifuged and washed with another volume of water, and resuspended in 1 volume of water. 1 mL samples of this cell suspension were treated by heating at 95°C for 0 (non-heated), 10, 20, 30, 60, 90 and 120 minutes, resulting in cell lysis, and the soluble and insoluble fractions were separated by centrifugation. The insoluble fraction was resuspended in 1 mL of buffer (50 mM Tris HCl pH 7.5, 300 mM NaCl, 10 mM MgCl2, 2 mM DTT, 0.5% Triton and Sigmafast Protease Inhibitor (Sigma)) and 10 µL aliquots of both soluble and insoluble fractions were analyzed by SDS-PAGE. In such conditions a 10 µL sample of soluble fraction and a 10 µL sample of insoluble fraction represent about the same amount of total cell extract. With no heating (0' on Figure 2), no cell lysis, or only residual cell lysis in water occurred. The pellet contains basically the entire cells, including the caseins, and this sample actually represents total cell extracts.

Surprisingly, il was observed that lysis by temperature impacted the distribution of caseins in the soluble and insoluble fractions (Figure 2).

By (or before) 10 minutes of heating at 95°C, alpha-S1 casein was almost entirely found in the soluble fraction (Figure 2A), whereas in example 1, it was found in both the soluble and soluble fraction in similar amounts.

Casein beta was progressively shifted to the soluble fraction, which contained large amount of this recombinant protein by (or before) 10 minutes of heating, and the major part by 30 minutes (Figure 2C). In example 1, beta casein was found mostly or entirely in the insoluble fraction.

The distribution of casein apha-S2 (entirely in the insoluble fraction in example 1) appeared to be less heat-sensitive, and it was still found in great part in the insoluble fraction by 120 minutes of heating (Figure 2B). Nevertheless, it appeared in the soluble fraction by 20 minutes of heating at 95°C, and was slightly decreasing in the insoluble fraction over time.

Many other protein bands from the insoluble fraction were diminishing as well over time (Figures 2A, 2B and 2C), but without being shifted into the soluble fraction. In the soluble fraction of the heated samples, only a few faint protein bands could be seen in addition to the casein bands.

These results indicate that lysis by heating is a good way to perform fast purification of caseins, and notably of alpha-S1 and beta casein.

### Example 3: Production of recombinant beta casein in a 42L fermenter (not according to the invention as defined by the claims)

A culture of recombinant strain described in Example 1 was used to inoculate a 42L fermenter (Biostat CPlus, Sartorius).

30L of LB medium supplemented with 30g/L of yeast extract (NuCel 751 MG) and 0.1 mg/L ampicillin were poured into the 42L fermenter. The fermenter was inoculated with a preculture of the recombinant clone, to reach an initial OD₆₀₀ of 0.06. The fermentation was conducted in a fed batch mode, at pH=7; T=37°C; pO₂=10%, with the culture being fed with a solution containing 143 g/L of D-Glucose and 214 g/L Yeast Extract. Ampicillin was added after 24 hours (0.1mg/L of culture). After 17 hours, the culture had reached an OD of about 10, and production was initiated by the addition of IPTG (1mM final). The culture was stopped at T=41,3, with OD₆₀₀=44. Cells were harvested by centrifugation, at 10°C.

For analysis, an aliquot of cells was centrifuged and resuspended in 1 volume of lysis buffer (50 mM Tris HCl pH 7.5, 300 mM NaCl, 10 mM MgCl2, 2 mM DTT, 0.5% Triton and Sigmafast Protease Inhibitor (Sigma). The suspension was incubated on ice for 30 min and then sonicated for 1 min (15% amplitude, Q Sonica XL-2000) and 10 µL of the total fraction, analyzed by SDS-PAGE (Figure 3A).

Purified beta casein from milk (Sigma) was used as control, but as observed previously by others displayed a higher apparent molecular weight (Simons et al. Overproduction of bovine beta-casein in Escherichia coli and engineering of its main chymosin cleavage site (1993) Protein Engineering 7:763-770).). Recombinant casein production was estimated to be in the range of 3 g/L of bacterial culture.

Samples were also analyzed for the presence of recombinant beta casein in the soluble and insoluble fractions, using the same two protocols as in Example 1, and results were similar with those observed in Example 1, with most (Protocol 1) or all (Protocol 2) of casein beta being found in the insoluble fraction (Figure 3B).

### Example 4: Monitoring of recombinant beta casein in the soluble and insoluble fraction of E. coli extracts resulting from heating at various temperatures

The impact of lysis by heating on recombinant beta casein was tested on strain culture described in Example 3.

1L of cells (about 15 g of dry cell weight) were centrifuged and cells were resuspended in 1L of sterile water, centrifuged and washed with another liter of water, and resuspended in 1L of water. 70 mL samples of this cell suspension were treated by heating at 95°C for 30, 60, 90 and 120 minutes, resulting in cell lysis, and the soluble and insoluble fractions were separated by centrifugation. The insoluble fraction was resuspended in 1 volume of lysis buffer (50 mM Tris HCl pH 7.5, 300 mM NaCl, 10 mM MgCl2, 2 mM DTT, 0.5% Triton and Sigmafast Protease Inhibitor (Sigma)) and 10 µL aliquots were analyzed by SDS-PAGE.

As shown on Figure 4A, the overexpressed protein was found mostly in the soluble fraction by (or before) 30 minutes of heating, in accordance with the results observed in Example 2 with thermal lysis, and in contrast with what was observed in Example 1 with other lysis protocols.

Many other protein bands from the insoluble fraction were diminishing as well over time, but without being shifted into the soluble fraction. In the soluble fraction of the heated samples, a few faint protein bands could be seen in addition to the beta casein band, but were also diminishing over time, a result that could barely be observed in Example 2, likely due to lower sample concentrations.

These results are consistent with the progressive heat degradation of lysate proteins observed in studies dedicated to the production and purification of thermophilic or thermostable proteins. (Takesawa et al. (1990) Heat-induced precipitation of cell homogenates: an investigation of the recovery of thermostable proteins. Enzyme Microb. Technol. 12, 184-189; Kirk and Cowan 1995 Optimising the recovery of recombinant thermostable proteins expressed in mesophilic hosts. J. of Biotechnology 42: 177-184; Sundarrajan et al. (2018) Novel properties of recombinant Sso7d-Taq DNA polymerase purified using aqueous two-phase extraction: Utilities of the enzyme in viral diagnosis. Biotechnol Rep (Amst)19:e00270; US8603782; WO2011119703 ).

The impact of different temperatures was also tested, i.e. 55°C, 75°C and 95°C on both the soluble and insoluble fractions, following the same protocol. As shown on Figure 4B, at 55°C a slight increase of beta casein amount in the soluble fraction could be observed by 120 minutes. At 75°C, a decrease of other soluble proteins was observed in the soluble (and insoluble fraction) by 1 hour, and the recovery of beta casein in the soluble fraction was more pronounced. At 95°C, beta casein was clearly observed in the soluble fraction by 10minutes of heating, and other bands were diminishing over time in both the insoluble and soluble fractions, with this last observation being clear in both fractions by 30 minutes of heating.

These results showed that lysis by heating is interesting for fast purification of beta casein, by both solubilizing intracellular casein and removing non casein proteins.

It also indicated that independently of lysis, heating could be used to isolate caseins from other, non-thermostable protein.

### Example 5: Raw purification of recombinant beta casein

A protocol was set for raw purification of casein beta from cells.

A cell sample corresponding to 15g of dry cell weight was resuspended in 1L of sterile water. Cells were treated by heating at 95°C for 90 minutes. The lysate was centrifuged at 3220 g and 4°C for 20 min, and the supernatant was filtered with a 0.2 µm membrane using a vacuum driven filtration system (Stericup Quick Release, Millipore).

Aliquots of samples from different steps were analyzed by SDS-PAGE. As shown on Figure 5, the final filtered protein fraction (lane F) was significantly enriched in recombinant protein, which eventually represented most of the protein in the preparation.

In addition, a protocol including thermal lysis can have a very good yield. In another experiment, a cell sample corresponding to 7,5 g of dry cell weight was washed and resuspended in of sterile water, and beta casein was monitored by SDS-PAGE, before and after heating for 90 minutes at 95°C. The amount of beta casein was found to be very similar in these two conditions, as well as in a filtered sample of the heated extract (Fig. 5B). Beta casein quantity was estimated by visual comparison with standard protein preparations, showing that about 3 g of recombinant protein per liter of culture could be observed in all three samples. Similar quantitative results were observed when samples from the same culture were heated for 30 minutes at 100°C and 2 bar pressure instead of 90 minutes at 95°C.

### Example 6: Making of a curd from partially purified beta casein

The casein solution described in Example 5 (Batch 1) was desiccated by heat. Analysis by SDS-PAGE were used to quantify the casein in this extract, and it was estimated that in 23% of the dessicated product corresponded to beta casein.

1.8 g of desiccated casein preparation (0,41g of casein) was mixed with 2.4g of water, and incubated at room temperature for 3.5 hours, for full rehydration. 3.5 g of deodorized coconut oil (BioPlanète, France) and 0.05g of lecithin were added, and pH was adjusted to 5 with 0.1g of lemon juice. The mixture was incubated for 30 minutes at 4°C for molding, and then removed from the mold at room temperature.

The product, whose final composition (but for chives) is summarized in Table 2 (composition 1), is featured on Figure 6A, showing a firm and stable texture at room temperature upon demolding, with perfect water retention. Casein content is estimated to be 5,3%.

This texture and water retention could not be achieved when the 1.8 g of desiccated casein preparation was not added (composition 2 on Table 2, Figure 6B). Instead, a solid phase was observed, due to coconut oil, but was forming crumbles upon demolding, and significant water loss was observed.

**Table 2. Various compositions**

| Composition number | 1 | 2 |
|---|---|---|
| Casein composition (g) | 1.8 | - |
| Water (g) | 2.4 | 4.2 |
| Coconut oil (g) | 3.5 | 3.5 |
| Lecithin (g) | 0.05 | 0.05 |
| Lemon juice (g) | 0.1 | 0.1 |
| Total (g) | 7.85 | 7.85 |

### Example 7: Purification of recombinant beta casein by heating and acid precipitation and monitoring of recombinant DNA

A sample of the culture described in example 3 was treated as described in example 5: cells were resuspended in sterile water, centrifuged, resuspended in water, the suspension was heated for 2 hours at 95°C, and the lysate supernatant was isolated and filtrated with a 0.2 µm membrane. Then, the supernatant was heated in acidic conditions, to precipitate caseins: the pH of the supernatant was decreased to pH 4 with a solution of 0.1 M of HCl and then heated at 40°C or 90°C for 20 min. The suspension was then centrifuged for 20 min at 3220 g, and the supernatant and pellets were analyzed by SDS-PAGE. As shown in Figure 7A, heating in such acidic conditions resulted in partial precipitation of casein beta at 40°C and full precipitation at 90°C.

In order to achieve a pH neutral solution, additional steps were added to this protocol: the pellet containing the precipitated casein was washed once with a solution of H₂SO₄ pH 4, once with sterile water, and resuspended in sterile H₂O and the pH Adjusted to 7 with 1 M Ca(OH)₂, resulting in a calcium caseinate suspension (not shown).

Heating in acidic conditions is known to promote DNA degradation. In order to assess the presence of DNA from the microbial production strain in the casein preparation, the same protocol was applied to another sample (but for the filtration step, which was skipped), and DNA was monitored using a PCR amplification. The reaction was performed using two primers designed to amplify a 929 bp region containing the beta casein coding sequence. Primer 1:
ATACATATGCGCGAGTTAGAAGAG (SEQ ID NO: 10)
Primer 2: CTTAATGCGCCGCTACAG (SEQ ID NO: 11)

PCR reaction was performed with 1 µL of sample in 20 µL using the DreamTaq Green PCR Master Mix (2X) (ThermoScientific). The PCR was performed using the SimpliAmp thermal Cycler (Applied Biosystems), and the cycling conditions were as follows: 95°C for 5 minutes, 30 cycles of the main reaction (95°C for 30 seconds, 61.4°C for 30 seconds, and 72°C for 1 minute), and 72°C for 5 minutes. The amplified products were loaded on a 1% agarose gel and visualized using the GeneFlash(Syngene) UV transilluminator. As shown on Figure 7B, recombinant DNA was still detectable under these conditions after thermolysis (lane A), and pH adjustment of the supernatant at pH 4 (lane B), but not detectable after heating at 90°C in such acidic conditions (lane C) and in the subsequent steps (lanes D and Ca).

### Example 8: Testing of the impact of a gelling agent on a fresh cheese substitute preparation (not according to the invention as defined by the claims) Mixing calcium caseinate, agar-agar, water, deodorized coconut oil and glucose:

18g of calcium caseinate casein (Armor Protéines, containing 92% caseins as caseinates; 1% lipids, and 1% calcium), were resuspended in 195 g of water. resulting in a 7.8% (w/w) casein in water suspension. Fifteen grammes of this casein composition were mixed with 2 g of deodorized coconut oil (BioPlanète, France) and 2.5 g of a 25% (w/w) glucose solution in water. The new composition was heated at 45°C, and 4 ml of a heated 0%, 2%, 3% or 4% agar-agar preparation (agar-agar melted in water) was added while gently mixing. Sample 1-4 were duplicated, as samples 5-8 (and samples 1, 4 and 8 were duplicated in a second, independent experiment). The resulting compositions (LpCC) are described in Table 4. pH is about 7.0.

**Table 4. Composition of the LpCC**

| | 1 | 2 | 3 | 4 | 5 | 6 | 7 | 8 |
|---|---|---|---|---|---|---|---|---|
| Total weight of LpCC | 23.5 g | 23.5 g | 23.5 g | 23.5 g | 23.5 g | 23.5 g | 23.5 g | 23.5 g |
| casein (w/w) | 5.0% | 5.0% | 5.0% | 5.0% | 5.0% | 5.0% | 5.0% | 5.0% |
| lipids (w/w) | 8.6% | 8.6% | 8.6% | 8.6% | 8.6% | 8.6% | 8.6% | 8.6% |
| glucose (w/w) | 2.7% | 2.7% | 2.7% | 2.7% | 2.7% | 2.7% | 2.7% | 2.7% |
| calcium (w/w) | 0.05% | 0.05% | 0.05% | 0.05% | 0.05% | 0.05% | 0.05% | 0.05% |
| agar-agar (w/w) | - | 0.34% | 0.51% | 0.68% | - | 0.34% | 0.51% | 0.68% |
| water (w/w) | 83.8% | 83.4% | 83.2% | 83.1% | 83.8% | 83.4% | 83.2% | 83.1% |
| Total (w/w) | 100.0% | 100.0% | 100.0% | 100.0% | 100.0% | 100.0% | 100.0% | 100.0% |
| water (fat-free basis) | 91.6% | 91.2% | 91.0% | 90.9% | 91.6% | 91.2% | 91.0% | 90.9% |

### Curdling

0.2 g lactic ferments (Streptococcus thermophilus, Lactobacillus bulgaricus, Alsa) were added in samples 1 to 4 (at an initial temperature of 25°C) but not in samples 5-8 (see Table 5) and the compositions were incubated at 40°C for 10 hours. In samples 1-4, pH dropped to about 4.5, indicating an active lactic fermentation. In samples 5-8, pH remained stably around 7.0.

### Draining

The entire composition was gently stirred, to disrupt the smooth structure obtained at the highest agar-agar concentrations and placed on a cheesecloth for draining at 4°C for 16 hours. Curd weights were ranging from about 7 g to about 15g as described in Table 5. Compositions were estimated (Table 5), based on the assumption that caseins, lipids and agar-agar were entirely retained in the curd. Indeed, little coloration was observed in the drained liquid for samples 1-4, and no coconut oil or agar-agar concretion were observed in the drained liquid at 4°C. In contrast, calcium and glucose estimates are maximal estimates, for part of these compounds may be found in the drained liquid. For the same reason, water content estimates (on a total basis and on a fat-free basis) are minimal estimates.

**Table 5. Compositions of curds**

| | 1 | 2 | 3 | 4* | 5 | 6 | 7 | 8 |
|---|---|---|---|---|---|---|---|---|
| Lactic ferment | + | + | + | + | - | - | - | |
| Curd solid phase weight | 7g* | 7g | 9g | 15g* | 2g | 7g | 7g | 12g* |
| casein (w/w) | 16.7% | 16.7% | 13.0% | 7.8% | ND | ND | ND | ND |
| lipids (w/w) | 28.8% | 28.8% | 22.4% | 13.4% | ND | ND | ND | ND |
| glucose (w/w) | 8.9% | 8.9% | 6.9% | 4.2% | ND | ND | ND | ND |
| calcium (w/w) | 0.18% | 0.18% | 0.14% | 0.08% | ND | ND | ND | ND |
| agar-agar (w/w) | | 1.1% | 1.3% | 1.1% | ND | ND | ND | ND |
| water (w/w) | 45.5% | 44.3% | 56.3% | 73.5% | ND | ND | ND | ND |
| Total (w/w) | 100.0% | 100.0% | 100.0% | 100.0% | ND | ND | ND | ND |
| water (fat-free basis) | 63.8% | 62.2% | 72.5% | 84.9% | ND | ND | ND | ND |

| | | | | | | | | |
|---|---|---|---|---|---|---|---|---|
| * average of two experiments | | | | | | | | |

The results show that the gelling agent can be used to modulate moisture, and therefore, the overall composition. With 0,68% agar-agar, more than 60% of the LpCC was retained (vs. 30% without gelling agent), and the product had the casein content and moisture of a fresh cheese, and a plastic texture and aspect, as expected (Figure 8).

In the absence of lactic fermentation, samples 5-8 gave solid phases of various sizes, depending an agar-agar concentration. The coloration of the drained liquid suggested that part of casein was lost from the solid phase, and estimates are therefore not indicated in Table 4. The solid phases obtained without ferment were smaller, as compared with their fermented curd counterpart (Table 4), but also kept a loose structure (Figure 8).

### Case of the addition of a gelling agent after curdling

The same effect could be achieved by adding the gelling agent after curdling: samples: LpCC with a similar composition was incubated for 10h at 40°C with lactic bacteria. pH dropped from about 7.0 to about 4.5. After curdling, 3 ml of agar-agar 4% in water was added, as described above, and the total mixture, including curd and liquid phase, was gently stirred, and placed on a cheesecloth for draining.

Compositions before curdling (LpCC), after curdling and agar-agar addition, and estimates after draining are indicated in Table 6, with the same provisions as above regarding composition after curdling and draining. After draining at 4°C for 16 hours, curds of about 12 g were obtained in two independent samples, showing a water retention (53% of total) superior to what is observed in the absence of gelling agent (see above).

The product had the casein content and moisture of a fresh cheese (Table 6), and a plastic texture and aspect, as expected (Figure 9). Thus, for certain types of cheese, which remain in a semi-solid form, it is possible to add it before or after curdling.

The procedure was replicated in the absence of ferment. A solid phase of lower weight (about 6 g, 27% of total) was obtained. However, texture was very loose, as shown on Figure 9.

**Table 6. Composition in process for fresh cheese substitute production**

| | LpCC | After curdling and agar-agar addition | Final after draining |
|---|---|---|---|
| weight (g) | 19.5g | 22.5g | 12g |
| casein (w/w) | 5.0% | 4.3% | 7.5% |
| lipids (w/w) | 10.3% | 8.9% | 15.4% |
| glucose (w/w) | 2.6% | 2.2% | 3.8% |
| calcium (w/w) | 0.05% | 0.05% | 0.08% |
| agar-agar (w/w) | - | 0.53% | 0.92% |
| water (w/w) | 82.1% | 83.9% | 72.3% |
| Total (w/w) | 100% | 100% | 100.0% |
| water (fat-free basis) | 91.5% | 92.2% | 85.5% |

### Example 9: Production of recombinant alpha and beta casein batch

Natural casein genes code for a precursor protein, which includes a signal peptide. In mammals, this peptide is cleaved during casein processing, and is not present in the mature protein in milk. As indicated in Example 1, synthetic genes coding for alpha-S1, and beta casein (related to natural genes P02662 and P02666, respectively), were modified, to remove the signal peptide, and the sequence of the new synthetic open reading frames are shown in Table 7, last column).

**Table 7. Sequences of natural caseins (precursors) and of the related recombinant proteins. In natural caseins, signal peptides are indicated in bold.**

| Name and Uniprot reference of protein precursor | sequence of protein precursor | sequence of recombinant proteins |
|---|---|---|
| Alpha-S1 casein P02662 | MKLLILTCLVAVALARPKHPIK HQGLPQEVLNENLLRFFVAPFP EVFGKEKVNELSKDIGSESTED QAMEDIKQMEAESISSSEEIVP NSVEQKHIQKEDVPSERYLGYL EQLLRLKKYKVPQLEIVPNSAE ERLHSMKEGIHAQQKEPMIGV NQELAYFYPELFRQFYQLDAY PSGAWYYVPLGTQYTDAPSFS | MRPKHPIKHQGLPQEVLNENLLR FFVAPFPEVFGKEKVNELSKDIG SESTEDQAMEDIKQMEAESISSS EEIVPNSVEQKHIQKEDVPSERY LGYLEQLLRLKKYKVPQLEIVPNS AEERLHSMKEGIHAQQKEPMIGV NQELAYFYPELFRQFYQLDAYPS GAWYYVPLGTQYTDAPSFSDIPN PIGSENSEKTTMPLW |
| | DIPNPIGSENSEKTTMPLW (SEQ ID NO: 1) | (SEQ ID NO: 2) |
| Beta casein P02666 | MKVLILACLVALALARELEELN VPGEIVESLSSSEESITRINKKIE KFQSEEQQQTEDELQDKIHPF AQTQSLVYPFPGPIPNSLPQNI PPLTQTPVVVPPFLQPEVMGV SKVKEAMAPKHKEMPFPKYPV EPFTESQSLTLTDVENLHLPLP LLQSWMHQPHQPLPPTVMFPP QSVLSLSQSKVLPVPQKAVPY PQRDMPIQAFLLYQEPVLGPV RGPFPIIV (SEQ ID NO: 7) | MRELEELNVPGEIVESLSSSEESI TRINKKIEKFQSEEQQQTEDELQ DKIHPFAQTQSLVYPFPGPIPNSL PQNIPPLTQTPVVVPPFLQPEVM GVSKVKEAMAPKHKEMPFPKYP VEPFTESQSLTLTDVENLHLPLPL LQSWMHQPHQPLPPTVMFPPQ SVLSLSQSKVLPVPQKAVPYPQR DMPIQAFLLYQEPVLGPVRGPFP IIV (SEQ ID NO: 8) |

The resulting ORFs were overexpressed in bacteria, and caseins were purified according to the methods described in the preceding examples. Eventually, three batches of animal-free recombinant caseins were obtained as calcium caseinates: to obtain concentrated calcium caseinates, casein extracts precipitated with H₂SO₄ at pH=4.6 were resuspended in water, pH was slowly adjusted to 7, with Ca(OH)₂, and the preparation was concentrated by evaporation using a Rotavapor R-300 (Buchi)] device.
Batch 1: beta casein (calcium caseinate), 150 mg/g of composition, pH7.
Batch 2: alpha-S1 casein and beta casein (calcium caseinate), about 50 mg/g and 25 mg/g, respectively, pH=7
Batch 3: alpha-S1 casein and beta casein (calcium caseinate), about 6 mg/g and 3 mg/g, respectively, pH=7

In these batches, other proteins and other organic molecules were potentially present, but caseins represented more than 90% of total proteins, and were estimated to represent more than 2/3 of dry weight.

### Example 10: Testing the curdling of partially purified recombinant beta casein,

Using batch 1 from example 9, a casein composition containing recombinant beta casein was used to test the curdling capacity of recombinant beta casein, in the absence of any texturing agent. For this, a preparation having approximatively the same casein, lipid, and water content as in sample 1 of example 8 was prepared, but with recombinant beta-casein instead of commercial caseinate.

Batch 1 preparation was diluted with water to obtain a recombinant beta-casein composition at a concentration of 58.7 mg/g. Twenty grammes of this casein composition was mixed with coconut oil, glucose, and agar-agar melted in water , to a 23.5g composition containing 5% casein, 8.5% lipids, 2.7% glucose and 0.68% agar-agar in water. Additional carbohydrate and proteins may come from the casein composition as well as salts and are not accounted for in this table. Therefore, water content is only an estimate as well, but given that in the initial casein composition, casein represented at least 2/3 of dry weight, errors in water content should therefore not exceed 3%.

The composition was seeded with 0.3 g lactic ferments and incubated at 40°C for 10 hours. pH dropped from about 7.0 to about 4.5. The entire composition was then placed on a cheesecloth for draining at 4°C for 16 hours. A curd of 7 g could be obtained after draining (Figure 10), although not as firm as the one obtained in similar conditions with commercial caseinates.

The ability of alpha-S1 and beta caseins from example 7 to coagulate efficiently at low pH was tested simply by the addition of lactic acid into 90 ml of the low concentration Batch 3 composition, to adjust pH at about 4.5. About 0,7g of material was obtained after separation by filtration on a cheese cloth (Figure 6). In contrast when no lactic acid was added to the same volume of composition, the entire composition flowed through the cheesecloth.

### Example 11: Making fresh cheese substitutes from partially purified recombinant caseins

Batch 1 preparation was diluted with water to obtain a recombinant beta-casein composition at a concentration of 78,2mg/g. Fifteen grammes of this casein composition was mixed with coconut oil, glucose, and agar-agar, as described in example 8, to obtain the composition described in Table 5. Additional carbohydrate and proteins may come from the casein composition as well as salts and are not accounted for in this table. Therefore, water content is only an estimate as well, but given that in the initial casein composition, casein represented at least 2/3 of dry weight, errors in water content should therefore not exceed 3%.

The composition was seeded with 0.3 g lactic ferments and incubated at 40°C for 10 hours. pH dropped from about 7.0 to about 4.5. The entire composition was gently stirred and placed on a cheesecloth for draining at 4°C for 16 hours.

Curd weight was about 14g. Its composition (Table 8) was estimated, with the same provisions as above and as in example 9. About 60% of the LpCC was retained. The resulting product had the casein content and moisture desired for fresh cheese. Texture was not as stiff as with commercial calcium caseinate in example 8, but nevertheless appropriate for a fresh cheese, displaying plasticity and firmness to be shaped in stable forms (Figure 11). For comparison, a sample was prepared following the same procedure, but lacking the lactic bacteria. In the absence of the curdling agent, a solid phase of about 13 g could be obtained, but with a loose structure, and it was not able to hold shapes stably (Figure 11).

The same procedure was followed, using Batch 2 of casein, containing apha-S1 casein and beta casein. Only 38% of the LpCC was retained, which could be due to the low protein content, (2.5% of LpCC), but the resulting product had the casein content and moisture desired for fresh cheese. Texture was not as stiff as with commercial calcium caseinate in example 8, but nevertheless appropriate for a fresh cheese, displaying plasticity and firmness to be shaped in stable forms (Figure 11).

**Table 8. Composition during process of making an edible composition**

| Nature of casein | Beta casein (Batch 1) | | Alpha-S1 and beta casein (Batch 2) | |
|---|---|---|---|---|
| Step | LpCC | final after curdling and draining | LpCC | final after curdling and draining |
| Weight (g) | 23 .5g | 14g | 23.5g | 9g |
| casein (w/w) | 5.0% | 8.4% | 2.5% | 6.4% |
| lipids (w/w) | 8.5% | 14.3% | 8.5% | 22.2% |
| glucose (w/w) | 2.7% | 4.5% | 2.7% | 6.9% |
| agar-agar (w/w) | 0.68% | 1.1% | 0.68% | 1.8% |
| water (w/w) | 83.1% | 71.7% | 85.7% | 62.6% |
| Total (w/w) | 100.0% | 100.0% | 100.0% | 100.0% |
| water (fat-free basis) | 90.9% | 83.7% | 93.7% | 80.5% |

### Case of the addition of a gelling agent after curdling

We also tested the addition of the gelling agent after curdling, following the same procedure as in example 8. Compositions before curdling (LpCC), after curdling and agar-agar addition, and estimates after draining are indicated in Table 9, with the same provisions as above regarding composition after curdling and draining. A curd of about 10 g (44% of total) was obtained after lactic fermentation, addition of agar-agar (0,53%), and draining at 4°C for 16 hours in a cheesecloth. In the absence of ferment, a solid phase of lower weight (about 6 g, 27% of total) was obtained. However, texture was very loose, as shown on Figure 12.

**Table 9. Composition during process of making an edible composition**

| | LpCC | After curdling and agar-agar addition | Final after draining |
|---|---|---|---|
| weight (g) | 19.5g | 22.5g | 10g |
| casein (w/w) | 5.0% | 4.3% | 9.0% |
| lipids (w/w) | 10.3% | 8.9% | 18.9% |
| glucose (w/w) | 2.6% | 2.2% | 4.7% |
| agar-agar (w/w) | - | 0.53% | 1.13% |
| water (w/w) | 82.2% | 83.9% | 66.3% |
| Total (w/w) | 100% | 100% | 100.0% |
| water (fat-free basis) | 91.6% | 92.2% | 81.7% |

### Example 12: Making a soft cheese substitute from partially purified recombinant caseins

Batch 1 preparation was further concentrated, to reach a concentration of 250mg/g of composition.

Fifteen grammes sunflower oil, 34 g of hydrated cashew nuts (for the composition of hydrated cashew nuts. see example 5 above), 70 g of water and 1.5 g of agar-agar were mixed. The mixture was brought to the boil and boiled for 10 to 20 seconds. One hundred grammes of the concentrated casein composition, containing 25 g of recombinant beta casein were added to this mixture and gently mixed. LpCC composition is detailed in Table 10.

### Curdling. draining. salting. and aging

200 g of this LpCC (out of a total of 220.5g) was seeded with ferments, including lactic bacteria (MBT, SOGEBUL, Dole, France), yeast (DH2d SOGEBUL, Dole, France) and additional ripening ferments (PC12H, SOGEBUL, Dole, France), at a temperature inferior to 35°C. The composition was incubated at 20°C for 24 hours, in a mold.

Product was removed from the mold, and set on a grid for draining. The product and grid were placed into a box to control hygrometry. About 1/4 to ½ teaspoon of table salt (La Baleine. France), depending on desired taste, can be spread on the upper face and the product is incubated at 14°C. Twenty-four hours later, the product is flipped, and the same salt composition is spread on the other face. The product is incubated at 14 °C on a grid into the refining box to control hygrometry, and flipped every second day, for several weeks. During this period, water is removed from the refining box on a regular manner. The product at day 7 is featured on Figure 13.

Weight loss consists essentially in water. Since the initial composition is known (with the composition of dry cashew nut being about 22% of carbohydrates. 20% of proteins. and 53% of lipids. and a few percent of water), an evaluation of water loss can be used for the calculation of the product composition, and for achieving the expected composition or a soft cheese, notably in terms of moisture (between 62% and 80% of moisture. on a fat free basis). By day 7, product was estimated to be 150g, protein and lipid content to be 17.4% (including 15.1% casein and derived peptides) and 16.6%, respectively. and moisture to be 76%. on a fat-free basis.

**Table 10. Composition of LpCC**

| | |
|---|---|
| weight (g) | 220.5g |
| proteins (w/w) | 13.1% |
| Including caseins | 11.3% |
| lipids (w/w) | 12.5% |
| glucose (w/w) | 1.2% |
| agar-agar (w/w) | 0.68% |
| water (w/w) | 72.5% |
| Total (w/w) | 100% |
| water (fat-free basis) | 82.9% |

### Example 13: Purification of recombinant alpha-S1 and beta caseins

A vector was constructed to co-express recombinant alpha-S1 and beta caseins. Basically, the ORFs coding for alpha-S1 and beta caseins (P02662 and P02666) from Table 1 were both inserted into pET25+, with the beta casein proximal to the promoter, and with a T7 ribosome binding site inserted before each one of the two ORFs. The resulting plasmid was transformed into BL21(DE3) strains. Individual transformed clones were isolated, and one clone was used to inoculate LB medium. Bacterial growth and casein expression was then conducted in a 42L fermenter (Biostat CPlus, Sartorius) as presented in Example 3.

At the end of fermentation, final OD was around 70. Cells were harvested by centrifugation (2000 g, 30 minutes, +4°C) and stored at -80°C. A clear yellow supernatant was obtained, having an OD between 0 to 1. After thawing, cells were resuspended in 15 L of osmosis sterile water, until having a homogeneous suspension, which was then heated during 120 minutes at 95°C. Cellular debris and *E.coli* precipitated proteins were discarded by (2000 g, 40 minutes, +4°C). The clear light-yellow supernatant, having an OD between 0 to 1, was stored at -20°C. After thawing, an additional high-speed centrifugation was carried out to remove residual cellular debris and precipitated proteins in suspension in the following conditions: 13000 g, +4°C, 20 minutes.

The clear light-yellow supernatant was stirred with commercial activated charcoal (Sigma-Aldrich, reference 161551) at a concentration of 10 g.L⁻¹, at 200 rpm and +22°C, during 30 minutes. Activated charcoal was then discarded through high-speed centrifugation (13000 g, 20 minutes, +4°C), and the supernatant was filtered on 0,2 µm filter, using a vacuum driven filtration system (Stericup Quick Release, Millipore). The clear beige supernatant was stored at +4°C during one night for practical reasons. The pH of the solution was then decreased to pH 4.6 with a commercial solution of lactic acid (Sigma-Aldrich, reference 27714). A white precipitate appeared gradually. The resulting suspension was centrifuged (2000 g, 20 minutes, RT) and the colorless and turbid supernatant was discarded. Acidic precipitated caseins were then resuspended in 7,5 L of osmosis sterile water and the resulting homogenous suspension was centrifuged (2000 g, 20 minutes, RT); the washing water was discarded. An additional wash step was performed in the same experimental conditions.

Acidic precipitated caseins were resuspended in 750 mL of osmosis sterile water. The resulting homogeneous suspension was stirred at 600 rpm, at +30°C and the pH was gradually raised to pH = 7.0, through the addition of a homogeneous calcium hydroxide suspension at a concentration of 0,2 M. The resulting suspension was then freezed at -80°C during 3 hours and then lyophilized (0,002 mbar, +4°C, 60 hours). Lyophilized calcium caseinates were stored at -20°C for further utilization or analysis.

Samples were analyzed by SDS PAGE at different steps of the process, as shown on Figure 14.

### Example 14: Making a soft cheese substitute from recombinant caseins alpha-S1 and beta

A composition of casein was prepared by co-expressing casein alpha-S1 and casein beta in a same bacteria. For this, open reading frames P02662 and P02666 from Table 1 were cloned in a same expression vector, transformed into *Escherichia coli,* produced in a fermenter and with protocol described in example 13 (using 0,45mm filtration instead of 0,2mm). Casein concentrations were estimated by SDS-PAGE. The preparation was estimated to have a purity of 95% at least, and to contain about half of alpha-S1 casein and half of beta casein. In all calculation below, this composition will be considered as pure (casein content should therefore 95% exact).

13 grammes of this dry casein composition was mixed with 39g of water, and incubated 3hours at 14°C. A mixture of water (15g), cashew nuts (20g), sunflower oil (30g), carbonate calcium (1g) and glucose (10g) was blended, and 22g of this mixture was added to 45g of the hydrated casein composition described above, together with 16 g of water, resulting in the LpCC described in Table 11.

**Table 1. Composition of LpCC**

| | |
|---|---|
| weight (g) | 83 g |
| proteins (w/w) | 14.3% |
| Including caseins | 13.5% |
| lipids (w/w) | 12.8 % |
| sugars (w/w) | 4.0% |
| Salts (w/w) | 0.5% |
| water (w/w) | 68.5% |
| Total (w/w) | 100% |
| water (fat-free basis) | 78.5% |

80 g of this composition was put in a mold seeded with ferments, including lactic bacteria (MBT, SOGEBUL, Dole, France), yeast (DH2d SOGEBUL, Dole, France) and additional ripening ferments (PC12H, SOGEBUL, Dole, France), and incubated 16h at a temperature of 50°C.

Product was then removed from the mold and set on a grid for draining. The product and grid were placed into a box to control hygrometry. Table salt (La Baleine. France) was spread on the upper face and the product was incubated at 14°C. Twenty-four hours later, the product was flipped, and the same salt composition was spread on the other face. The product was incubated at 14 °C on a grid into the refining box to control hygrometry, and flipped every second day, for 6 days, and then kept at 4°C. Product weight was estimated to be 60g, with the composition described in Table 12. This composition is also taking into account degradation products, resulting from lipolysis, proteolysis and sugar metabolization resulting from the activity of starter bacteria and ripening ferments) for each category. Moisture was estimated to be 68.5%. on a fat-free basis. The product at day 6 is featured on Figure 15.

**Table 2. Final composition of soft cheese (Salts do not include added table salt)**

| | |
|---|---|
| weight (g) | 60 g |
| proteins (w/w) | 19.7% |
| Including caseins | 18.8% |
| lipids (w/w) | 17.7% |
| sugars (w/w) | 5.5% |
| Salts (w/w) | 0.7% |
| water (w/w) | 56.3% |
| Total (w/w) | 100% |
| water (fat-free basis) | 68.5% |

## Claims

1. A method for the enrichment of casein in a composition containing casein and other proteins, comprising:
i) providing a composition comprising microorganisms that have been transformed with at least one nucleic acid coding for a casein, and the microorganisms have been cultured so as to express and produce casein, wherein the microorganisms express other proteins, wherein the pH of the composition is equal or above 6.5 and preferably below 9
ii) heating the composition so as to reduce the amount of the other proteins in soluble fraction of the composition, wherein heating is performed at a temperature comprised between 75°C and 105°C
iii) recovering the soluble fraction,
thereby obtaining a soluble fraction enriched in casein.

2. A method for the production of a composition of casein comprising:
i) providing a microorganism composition, wherein the microorganism composition comprises microorganisms that have been transformed with at least one nucleic acid coding for a casein, and the microorganisms have been cultured so as to express and produce casein, wherein the pH of the composition is equal or above 6.5 and preferably below 9
ii) heating the microorganism composition so as to lyse the microorganisms cells and to reduce the amount of the other proteins in the soluble fraction of the composition, wherein heating is performed at a temperature comprised between 75°C and 105°C
iii) isolating the soluble fraction from the lysed cell composition
thereby obtaining a composition of casein.

3. The method of claim 1 or 2, wherein heating in ii) is performed for at least one hour.

4. The method of any one of claims 1 to 3, wherein heating in ii) increases the ratio of casein in the soluble fraction.

5. The method of any one of claims 1 to 4, wherein the microorganisms in the microorganism composition are not lysed and heating in ii) lyses the microorganisms.

6. The method of claim 5, wherein heating in ii) is performed at a temperature of about 95°C.

7. The method of any one of claims 1 to 6, wherein heating in ii) is performed for at least one hour.

8. The method of any one of claims 1 to 7, wherein the microorganisms have been transformed with one or more nucleic acid coding for more than one casein.

9. The method of any one of claims 1 to 8, wherein the casein is selected from beta casein, alpha-S1 casein, alpha-S2 casein and mixture of these caseins.

10. The method of any one of claims 1 to 8, wherein the casein is selected from beta casein, alpha-S1 casein and mixture of these caseins.

11. The method of any one of claims 1 to 10, further comprising a step of performing a further purification of casein of the soluble fraction obtained in iii).

12. The method of claim 11, wherein the further step applied to the soluble fraction comprises at least one step selected in the group consisting of addition of activated charcoal, membrane filtration, chromatography and casein precipitation.

13. The method of claim 12, wherein the further step is acidic precipitation of casein.

14. The method of claim 13, wherein the further step of acidic precipitation of casein is conducted by heating at 90°C at pH=4.

15. The method of any one of claims 1 to 14, wherein the microorganism composition is a bacterial composition.

## Patentansprüche

1. Verfahren zur Anreicherung von Kasein in einer Zusammensetzung, die Kasein und andere Proteine enthält, umfassend:
i) Bereitstellen einer Zusammensetzung, die Mikroorganismen umfasst, die mit mindestens einer für ein Kasein kodierenden Nukleinsäure transformiert wurden, und wobei die Mikroorganismen so kultiviert wurden, dass sie Kasein exprimieren und produzieren, wobei die Mikroorganismen andere Proteine exprimieren, wobei der pH-Wert der Zusammensetzung 6,5 oder mehr und vorzugsweise unter 9 beträgt
ii) Erhitzen der Zusammensetzung, um die Menge der anderen Proteine in der löslichen Fraktion der Zusammensetzung zu verringern, wobei das Erhitzen bei einer Temperatur zwischen 75 °C und 105 °C durchgeführt wird
iii) Gewinnen der löslichen Fraktion,
wodurch eine mit Casein angereicherte lösliche Fraktion erhalten wird.

2. Verfahren zur Herstellung einer Casein-Zusammensetzung, umfassend:
i) Bereitstellen einer Mikroorganismen-Zusammensetzung, wobei die Mikroorganismen-Zusammensetzung Mikroorganismen umfasst, die mit mindestens einer für ein Casein kodierenden Nukleinsäure transformiert wurden, und die Mikroorganismen so kultiviert wurden, dass sie Casein exprimieren und produzieren, wobei der pH-Wert der Zusammensetzung 6,5 oder mehr und vorzugsweise unter 9 beträgt
ii) Erhitzen der Mikroorganismenzusammensetzung, um die Mikroorganismenzellen zu lysieren und die Menge der anderen Proteine in der löslichen Fraktion der Zusammensetzung zu verringern, wobei das Erhitzen bei einer Temperatur zwischen 75 °C und 105 °C durchgeführt wird
iii) Isolieren der löslichen Fraktion aus der lysierten Zellzusammensetzung wodurch eine Casein-Zusammensetzung erhalten wird.

3. Verfahren nach Anspruch 1 oder 2, wobei das Erhitzen in ii) mindestens eine Stunde lang durchgeführt wird.

4. Verfahren nach einem der Ansprüche 1 bis 3, wobei das Erhitzen in ii) den Anteil an Kasein in der löslichen Fraktion erhöht.

5. Verfahren nach einem der Ansprüche 1 bis 4, wobei die Mikroorganismen in der Mikroorganismenzusammensetzung nicht lysiert sind und das Erhitzen in ii) die Mikroorganismen lysiert.

6. Verfahren nach Anspruch 5, wobei das Erhitzen in ii) bei einer Temperatur von etwa 95 °C durchgeführt wird.

7. Verfahren nach einem der Ansprüche 1 bis 6, wobei das Erhitzen in ii) mindestens eine Stunde lang durchgeführt wird.

8. Verfahren nach einem der Ansprüche 1 bis 7, wobei die Mikroorganismen mit einer oder mehreren Nukleinsäuren transformiert wurden, die für mehr als ein Casein kodieren.

9. Verfahren nach einem der Ansprüche 1 bis 8, wobei das Casein aus Beta-Casein, Alpha-S1-Casein, Alpha-S2-Casein und einer Mischung dieser Caseine ausgewählt ist.

10. Verfahren nach einem der Ansprüche 1 bis 8, wobei das Casein aus Beta-Casein, Alpha-S1-Casein und einer Mischung dieser Caseine ausgewählt ist.

11. Verfahren nach einem der Ansprüche 1 bis 10, das ferner einen Schritt zur Durchführung einer weiteren Reinigung des in iii) erhaltenen Caseins der löslichen Fraktion umfasst.

12. Verfahren nach Anspruch 11, wobei der auf die lösliche Fraktion angewandte weitere Schritt mindestens einen Schritt umfasst, der aus der Gruppe ausgewählt ist, bestehend aus Zugabe von Aktivkohle, Membranfiltration, Chromatographie und Caseinausfällung.

13. Verfahren nach Anspruch 12, wobei der weitere Schritt eine saure Ausfällung von Casein ist.

14. Verfahren nach Anspruch 13, wobei der weitere Schritt der sauren Ausfällung von Casein durch Erhitzen auf 90 °C bei einem pH-Wert von 4 durchgeführt wird.

15. Verfahren nach einem der Ansprüche 1 bis 14, wobei die Mikroorganismenzusammensetzung eine bakterielle Zusammensetzung ist.

## Revendications

1. Méthode d'enrichissement en caséine d'une composition contenant de la caséine et d'autres protéines, comprenant :
i) la mise à disposition d'une composition comprenant des micro-organismes qui ont été transformés avec au moins un acide nucléique codant pour une caséine, et dont les micro-organismes ont été cultivés de manière à exprimer et à produire de la caséine, dans lequel les micro-organismes expriment d'autres protéines, et dans lequel le pH de la composition est égal ou supérieur à 6,5 et de préférence inférieur à 9
ii) le chauffage de la composition de manière à réduire la quantité des autres protéines dans la fraction soluble de la composition, le chauffage étant effectué à une température comprise entre 75 °C et 105 °C
iii) la récupération de la fraction soluble,
une fraction soluble enrichie en caséine étant ainsi obtenue.

2. Méthode de production d'une composition de caséine comprenant :
i) la mise à disposition d'une composition de micro-organismes, dans laquelle la composition de micro-organismes comprend des micro-organismes qui ont été transformés avec au moins un acide nucléique codant pour une caséine, et les micro-organismes ont été cultivés de manière à exprimer et à produire de la caséine, le pH de la composition étant égal ou supérieur à 6,5 et de préférence inférieur à 9
ii) le chauffage de la composition de micro-organismes de manière à lyser les cellules des micro-organismes et à réduire la quantité des autres protéines dans la fraction soluble de la composition, le chauffage étant effectué à une température comprise entre 75 °C et 105 °C
iii) l'isolation de la fraction soluble de la composition de cellules lysées une composition de caséine étant ainsi obtenue.

3. Méthode selon la revendication 1 ou 2, dans lequel le chauffage en ii) est effectué pendant au moins une heure.

4. Méthode selon l'une quelconque des revendications 1 à 3, dans lequel le chauffage en ii) augmente la proportion de caséine dans la fraction soluble.

5. Méthode selon l'une quelconque des revendications 1 à 4, dans lequel les micro-organismes présents dans la composition de micro-organismes ne sont pas lysés et le chauffage en ii) lyse les micro-organismes.

6. Méthode selon la revendication 5, dans lequel le chauffage en ii) est effectué à une température d'environ 95 °C.

7. Méthode selon l'une quelconque des revendications 1 à 6, dans lequel le chauffage en ii) est effectué pendant au moins une heure.

8. Méthode selon l'une quelconque des revendications 1 à 7, dans lequel les micro-organismes ont été transformés avec un ou plusieurs acides nucléiques codant pour plus d'une caséine.

9. Méthode selon l'une quelconque des revendications 1 à 8, dans lequel la caséine est choisie parmi la bêta-caséine, l'alpha-S1-caséine, l'alpha-S2-caséine et un mélange de ces caséines.

10. Méthode selon l'une quelconque des revendications 1 à 8, dans lequel la caséine est choisie parmi la bêta-caséine, l'alpha-S1-caséine et un mélange de ces caséines.

11. Méthode selon l'une quelconque des revendications 1 à 10, comprenant en outre une étape consistant à effectuer une purification supplémentaire de la caséine de la fraction soluble obtenue en iii).

12. Méthode selon la revendication 11, dans lequel l'étape supplémentaire appliquée à la fraction soluble comprend au moins une étape choisie dans le groupe constitué par l'ajout de charbon actif, la filtration sur membrane, la chromatographie et la précipitation de la caséine.

13. Méthode selon la revendication 12, dans lequel l'étape supplémentaire consiste en une précipitation acide de la caséine.

14. Méthode selon la revendication 13, dans lequel l'étape supplémentaire de précipitation acide de la caséine est réalisée par chauffage à 90 °C à un pH = 4.

15. Méthode selon l'une quelconque des revendications 1 à 14, dans lequel la composition de micro-organismes est une composition bactérienne.
